(19) Europäisches Patentamt
European Patent Office
Office européen des brevets

(11) Veröffentlichungsnummer: 0 374 756
A2

(12) # EUROPÄISCHE PATENTANMELDUNG

(21) Anmeldenummer: 89123237.3

(22) Anmeldetag: 15.12.89

(51) Int. Cl.5: C07D 217/14, A61K 31/47, C07D 409/12, C07D 209/44, C07D 401/12, C07D 213/36, C07D 295/12

(30) Priorität: 23.12.88 DE 3843469
24.10.89 DE 3935371

(43) Veröffentlichungstag der Anmeldung: 27.06.90 Patentblatt 90/26

(84) Benannte Vertragsstaaten: AT BE CH DE ES FR GB IT LI NL SE

(71) Anmelder: MERCK PATENT GESELLSCHAFT MIT BESCHRÄNKTER HAFTUNG
Frankfurter Strasse 250 Postfach 4119
D-6100 Darmstadt(DE)

(72) Erfinder: Gottschlich, Rudolf, Dr.
Buchenweg 1
D-6107 Reinheim(DE)

Erfinder: Prücher, Helmut
Königsbergerstrasse 9
D-6148 Heppenheim(DE)
Erfinder: Ackermann, Karl-August
Am Pfarrweiher 40
D-6105 Ober-Ramstadt(DE)
Erfinder: Barber, Andrew, Dr.
Soderstrasse 55
D-6100 Darmstadt(DE)
Erfinder: Greiner, Hartmut, Dr.
Dieburger Strasse 218
D-6100 Darmstadt(DE)
Erfinder: Haase, Anton, Dr.
Ahornweg 5
D-6109 Mühltal(DE)

(54) **Stickstoffhaltige Ringverbindungen.**

(57) Stickstoffhaltige Ringverbindungen der Formel I

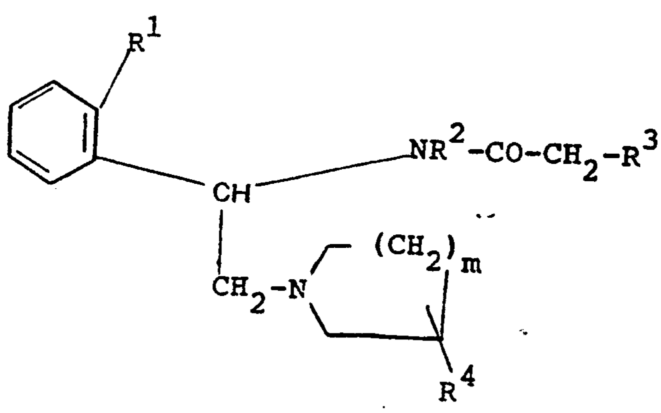

I

worin $R^1$, $R^2$, $R^3$, $R^4$ und m die in Patentanspruch 1 angegebenen Bedeutung haben, sowie deren Salze zeigen analgetische Wirkungen.

EP 0 374 756 A2

## Stickstoffhaltige Ringverbindungen

Die Erfindung betrifft neue stickstoffhaltige Ringverbindungen der Formel I

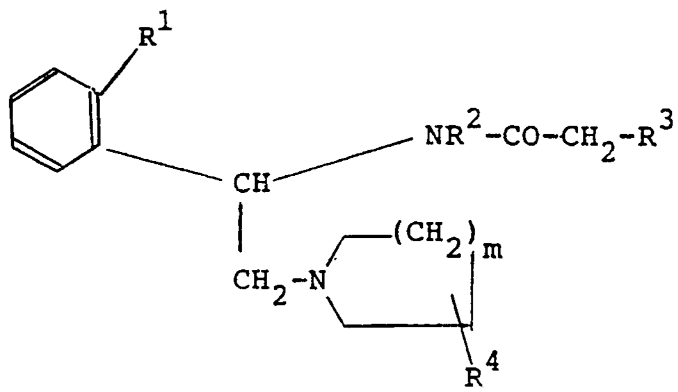

I

worin

$R^1$ H,

$R^2$ A,

$R^1$ und $R^2$ zusammen auch $-(CH_2)_n-$,

$R^3$ eine Phenyl-, Thienyl-, Naphthyl- oder Benzothienylgruppe,

$R^4$ H oder E-Q und

m und n jeweils 1, 2 oder 3

bedeuten,

worin ferner im Benzolring und/oder im Rest $R^3$ unabhängig voneinander jeweils eine CH-Gruppe durch N ersetzt sein kann (können) und/oder worin der Benzolring und/oder der Rest $R^3$ unabhängig voneinander jeweils ein-, zwei- oder dreifach durch A, OA, F, Cl, Br, I, $CF_3$, $NO_2$, $NH_2$, NHA, $NA_2$ und/oder jeweils durch eine Methylendioxygruppe und/oder jeweils durch eine oder zwei der Gruppen -NZ-CY-NZ-und/oder -E-Q substituiert sein kann (können),

worin

E fehlt oder -alkyl-, -O-alkyl-, -NZ-alkyl-, -CY-alkyl-, -NZ-CY-alkyl- oder -CY-NZ-alkyl-,

Q -OH, -NZ-CY-Z, $NZ-(CY)_r-NHZ$, $-NZ-SO_2-A$, $-COOR^5$, -CO-NH-Z,

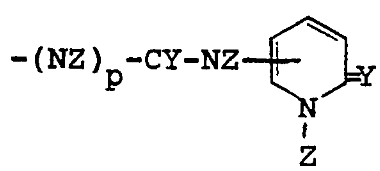

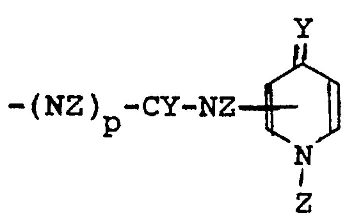,

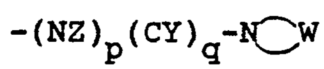 oder $-SO_2-NHZ$,

W $-CH_2CH_2-NZ-CY-$ oder $-CT^1-CT^2-NZ-CT^3-CT^4-$,

Y =O oder =NZ,

Z H oder A,

$T^1$, $T^2$, $T^3$ und $T^4$ jeweils =O oder (H, H),

$R^5$ H, A, AO-alkyl, A-CO-O-alkyl, AO-CO-O-alkyl oder 2 $-R^6O-3-R^7O$-propyl,

$R^6$ und $R^7$ jeweils A oder zusammen Alkyliden oder Cycloalkyliden mit jeweils bis zu 6-Atomen,

p und q 0 oder 1,

r 1 oder 2,

A Alkyl mit 1-6 C-Atomen und

-alkyl Alkylen mit 1-4 C-Atomen bedeuten,
worin ferner, falls mehrere Gruppen A, E, Q, Y und/oder Z vorhanden sind, diese jeweils gleich oder verschieden sein können,
sowie deren Salze.

Ähnliche Verbindungen sind in der DE-A-27 49 950 beschrieben.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze wertvolle pharmakologische Eigenschaften besitzen. Insbesondere zeigen sie analgetische Eigenschaften. So wirken die Verbindungen besonders stark im "Writhing Test" an Mäusen oder Ratten (Methode vgl. Siegmund et al., Proc. Soc. Exp. Biol. 95, (1957), 729-731). Die analgetische Wirkung läßt sich ferner im "Tail-Flick-Test" an Mäusen oder Ratten nachweisen (Methodik vgl. d'Amour und Smith, J. Pharmacol. Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur). Dabei zeigen die Verbindungen keine oder nur geringe Neigung zu physischer Abhängigkeit. Außerdem treten antiinflammatorische, antiasthmatische, diuretische, antikonvulsive und/oder antitussive Wirkungen auf, die ebenfalls nach hierfür geläufigen Methoden nachgewiesen werden können. Die Verbindungen eignen sich ferner zum Schutz vor und zur Behandlung von Hirnödemen und Unterversorgungszuständen des Zentralnervensystems, vor allem Hypoxie.

Die Verbindungen können daher als Arzneimittelwirkstoffe in der Human- und Veterinärmedizin verwendet werden. Ferner eignen sie sich als Zwischenprodukte zur Herstellung anderer Verbindungen mit wertvollen Eigenschaften.

Gegenstand der Erfindung sind Verbindungen der Formel I sowie ihre Salze.

Die Gruppe A steht vorzugsweise für Alkyl mit 1, 2, 3 oder 4 C-Atomen, insbesondere für Methyl oder Ethyl, aber auch für Propyl, Isopropyl, Butyl, sek.-Butyl, tert. -Butyl, Pentyl oder Hexyl. Die Gruppe OA ist dementsprechend vorzugsweise Methoxy oder Ethoxy, ferner Propoxy, Isopropoxy, Butoxy, Isobutoxy, sek.-Butoxy, tert.-Butoxy, Pentyloxy oder Hexyloxy.

Die Gruppe "-alkyl" stheht vorzugsweise für $-(CH_2)_t-$(t = 1, 2, 3, 4, 5 oder 6), insbesondere für $-CH_2-$ oder $-(CH_2)_2-$, aber auch bevorzugt für $-(CH_2)_3-$, $-(CH_2)_4-$, $-(CH_2)_5-$, $-(CH_2)_6-$, $-CH(CH_3)-$, $-C(CH_3)_2-$, $-CH(CH_3)-CH_2-$, $-CH_2-CH(CH_3)-$ oder $-CH_2-C(CH_3)_2-$.

Die Verbindungen der Formel I, worin $R^1$ und $R^2$ zusammen $-(CH_2)_n-$ bedeuten und worin im Benzolring keine CE-Gruppe durch N ersetzt ist, umschließen im einzelnen die entsprechenden Verbindungen der Teilformeln Ia bis Ii (worin die Reste $R^3$ und $R^4$ die bei Formel I angegebenen Bedeutungen haben):

Ia 1-Pyrrolidinomethyl-isoindoline (I, m = n = 1);
Ib 1-Piperidinomethyl-isoindoline (I, m =2, n = 1);
Ic 1-Hexahydroazepinomethyl-isoindoline (I, m = 3, n = 1);
Id 1-Pyrrolidinomethyl-1,2,3,4-tetrahydroisochinoline (I, m = 1, n = 2);

Ie 1-Piperidinomethyl-1,2,3,4-tetrahydroisochinoline (I, m = n = 2);
If 1-Hexahydroazepinomethyl-1,2,3,4-tetrahydroisochinoline (I, m = 3, n = 2);
Ig 1-Pyrrolidinomethyl-2,3,4,5-tetrahydro-1H-2-benzazepine (I, m = 1, n = 3);
Ih 1-Piperidinomethyl-2,3,4,5-tetrahydro-1H-2-benzazepine (I, m = 2, n = 3);
Ii-Hexahydroazepinomethyl-2,3,4,5-tetrahydro-1H-2-benzazepine (I, m = n = 3).

Verbindungen der Formel I, worin $R^1$ H und $R^2$ A ist, umschließen im einzelnen die entsprechenden Verbindungen der Teilformeln Ij bis Io (worin die Reste $R^3$ und $R^4$ die bei Formel I angegebenen Bedeutungen haben:

Ij N-[2-Phenyl-2-($NR^2$-$COCH_2R^3$)-ethyl]-pyrrolidine (m = 1),
Ik N-[2-(Pyridyl)-2-($NR^2$-$COCH_2R^3$)-ethyl]-pyrrolidine (m = 1),
Il N-[2-Phenyl-2-($NR^2$-$COCH_2R^3$)-ethyl]-piperidine (m = 2),
Im N-[2-(Pyridyl)-2-($NR^2$-$COCH_2R^3$)-ethyl]-piperidine (m = 2),
In N-[2-Phenyl-2-($NR^2$-$COCH_2R^3$)-ethyl]-hexahydroazepine (m = 3)
Io N-[2-(Pyridyl)-2-($NR^2$-$COCH_2R^3$)-ethyl]-hexahydroazepine (m = 3)

In den Verbindungen Ij bis Io ist $R^2$ vorzugsweise Methyl.

Die Verbindungen der Formeln Id, Ij und Ik, ferner diejenigen der Formeln Ie, Il und Im sind bevorzugt; n ist also vorzugsweise 1, ferner 2, n ist vorzugsweise 2.

Z ist vorzugsweise H, ferner bevorzugt $CH_3$.

Y ist vorzugsweise =O, ferner bevorzugt -NH oder $=NCH_3$.

Von den vier Gruppen $T^1$, $T^2$, $T^3$ und $T^4$ sind bevorzugt keine, eine oder zwei =O, die übrigen (H,H).

W ist vorzugsweise $-CH_2CH_2-NH-CO-$, ferner bevorzugt $-CH_2CH_2-N(CH_3)-CO-$, $-CH_2CH_2-NH-CH_2CH_2-$,

$-CH_2CH_2-N(CH_3)-CH_2CH_2-$, $-CO-CH_2-NH-CH_2CH_2-$ oder $-CO-CH_2-NH-CO-CH_2-$.

Dementsprechend ist E vorzugsweise fehlend oder -alkyl-, ferner bevorzugt -O-alkyl-, -NH-alkyl-, -CO-alkyl-, -NH-CO-alkyl- oder -CO-NH-alkyl-.

Q ist vorzugsweise -OH, -NH-CO-Z wie -NH-CO-H oder $-NH-CO-CH_3$, $-NH-CO-NH_2$, $-NH-CO-NHCH_3$, $-NH-CO-CO-NH_2$, $-NH-SO_2A$ wie $-NH-SO_2CH_3$, $-COOR^5$ wie COOH oder $COOCH_3$, $-CONH_2$ oder $-CO-NH-CH_3$.

Die Gruppe E-Q ist vorzugsweise $-NH-CO-NH_2$, $-CONH_2$ oder $-CH_2CONH_2$, ferner bevorzugt -O-alkyl-CONHZ wie $-O-CH_2CONH_2$ oder $-O-CH_2CONHCH_3$, -O-alkyl-COOZ wie $-O-CH_2COOH$, $-O-CH_2-COOCH_3$ oder $-O-CH_2-COOC_2H_5$, -NZCO-Z wie -NH-CO-H, $-NH-CO-CH_3$, $-N(CH_3)-CO-H$ oder $-N(CH_3)-CO-CH_3$, $-NHSO_2A$ wie $-NHSO_2CH_3$, -NH-CO-NH-A wie $-NH-CO-NH-CH_3$, $-NH-CO-CO-NH_2$, -COOZ wie -COOH, $-COOCH_3$ oder $-COOC_2H_5$, -alkyl-COOZ wie $-CH_2-COOH$, $-CH_2-COOCH_3$ oder $-CH_2-COOC_2H_5$ oder CONHA wie $-CO-NH-CH_3$, weiterhin bevorzugt -OH; -alkyl-OH wie $-CH_2OH$, $-CH(CH_3)-OH$, $-CH_2CH_2OH$, $-C-(CH_3)_2-OH$, $-CH_2-CH(CH_3)-OH$, $-CH_2-C(CH_3)_2OH$; -O-alkyl-OH wie $-O-CH_2CH_2-OH$; -NZ-alkyl-OH wie $-NH-CH_2\ CH_2-OH$; -NZ-CO-alkyl-OH wie $-NH-CO-CH_2CH_2-OH$ oder $-N(CH_3)-CO-CH_2CH_2-OH$; -CO-NZ-alkyl-OH wie $CO-NH-CH_2CH_2-OH-$; -alkyl-NZ-CO-Z wie $-CH_2-NH-CO-H$, $-CH_2NH-COCH_3$, $-CH_2CH_2-NH-CO-H$ oder $-CH_2CH_2-NH-COCH_3$; -O-alkyl-NZ-CO-Z wie $-O-CH_2CH_2-NH-CO-H$ oder $-O-CH_2CH_2-NH-COCH_3$; -NZ-alkyl-NZ-CO-Z wie $-NH-CH_2CH_2-NH-CO-H$, $-NH-CH_2CH_2-NH-COCH_3$, $-N(CH_3)-CH_2CH_2-NH-CO-H$ oder $-N(CH_3)-CH_2CH_2-NH-COCH_3$; -CO-alkyl-NZ-CO-Z wie $-CO-CH_2-NH-CO-H$, $-CO-CH_2-NH-COCH_3$, $-CO-CH_2CH_2-NH-CO-H$ oder $-CO-CH_2CH_2-NH-COCH_3$; -NZ-CO-alkyl-NZ-CO-Z wie $-NH-CO-CH_2-NH-CO-H$, $-NH-CO-CH_2-NH-COCH_3$, $-N(CH_3)-CO-CH_2-NH-CO-H$, $-N(CH_3)-CO-CH_2-NH-COCH_3$, $-NH-CO-CH_2CH_2-NH-CO-H$, $-NH-CO-CH_2CH_2-NH-COCH_3$, $-N(CH_3)-CO-CH_2CH_2-NH-CO-H$ oder $-N(CH_3)-CO-CH_2-CH_2-NH-COCH_3$; -CO-NZ-alkyl-NZ-CO-Z wie $-CO-NH-CH_2CH_2-NH-CO-H$ oder $-CO-NH-CH_2CH_2-NH-COCH_3$; -alkyl-NZ-CO-NHZ wie $-CH_2-NH-CO-NH_2$ oder $-CH_2CH_2-NH-CO-NH_2$; -O-alkyl-NZ-CO-NHZ wie $-O-CH_2CH_2-NH-CO-NH_2$ oder $-O-CH_2CH_2-NH-CO-NH-CH_3$; -NZ-alkyl-NZ-CO-NHZ wie $-NH-CH_2CH_2-NH-CO-NH_2$, $-NH-CH_2CH_2-NH-CO-NH-CH_3$, $-N(CH_3)-CH_2CH_2-NH-CO-NH_2$ oder $-N(CH_3)-CH_2CH_2-NH-CO-NH-CH_3$; -CO-alkyl-NZ-CO-NHZ wie $-CO-CH_2-NH-CO-NH_2$, $-CO-CH_2-NH-CO-NH-CH_3$,$-CO-CH_2CH_2-NH-CO-NH_2$ oder $-CO-CH_2CH_2-NH-CO-NH-CH_3$; -NZ-CO-alkyl-NZ-CO-NHZ wie $-NH-CO-CH_2-NH-CO-NH_2$, $-NH-CO-CH_2CH_2-NH-CO-NH_2$, $-N(CH_3)-CO-CH_2-NH-CO-NH_2$ oder $-N(CH_3)-CO-CH_2CH_2-NH-CO-NH_2$; -CO-NZ-alkyl-NZ-CO-NHZ wie $-CO-NH-CH_2CH_2-NH-CO-NH_2$ oder $-CO-NH-CH_2CH_2-NH-CO-NH-CH_3$; $-alkyl-NH-SO_2-A$ wie $-CH_2-NH-SO_2-CH_3$ oder $-CH_2CH_2-NH-SO_2-CH_3$; $-O-alkyl-NH-SO_2-A$ wie $-O-CH_2CH_2-NH-SO_2-CH_3$ ; $-NZ-alkyl-NH-SO_2-A$ wie $-NH-CH_2CH_2-NH-SO_2-CH_3$ oder $-N(CH_3)-CH_2CH_2-NH-SO_2-CH_3$; $-NZ-CO-alkyl-NH-SO_2-A$ wie $-NH-CO-CH_2CH_2-NH-SO_2CH_3$; $-CO-NZ-alkyl-NH-SO_2-A$ wie $-CO-NH-CH_2CH_2-NH-SO_2-CH_3$; -NZ-alkyl-COOZ wie $-NH-CH_2-COOH$, $-NH-CH_2-COOCH_3$, $-NH-CH_2CH_2-COOH$ oder $-NH-CH_2CH_2-COOCH_3$; -CO-alkyl-COOZ wie $-CO-CH_2CH_2-COOH$ oder $-CO-CH_2CH_2-COOCH_3$; -NZ-CO-alkyl-COOZ wie $-NH-CO-CH_2CH_2-COOH$ oder $-NH-CO-CH_2CH_2-COOC_2H_5$; -CO-NZ-alkyl-COOZ wie $-CO-NH-CH_2-COOH$ oder $-CO-NH-CH_2CH_2-COOCH_3$; -alkyl-CO-NHZ wie $-CH_2-CO-NHCH_3$, $-CH_2CH_2-CONH_2$ oder $-CH_2CH_2-CO-NHCH_3$; $-O-CH_2CH_2-CO-NHZ$ wie $-O-CH_2CH_2-CONH_2$ oder $-O-CH_2CH_2-CO-NHCH_3$; -NZ-alkyl-CO-NHZ wie $-NH-CH_2-CONH_2$, $-NH-CH_2-CO-NHCH_3$, $-N(CH_3)-CH_2-CONH_2$, $-N(CH_3)-CH_2-CO-NHCH_3$, $-NH-CH_2CH_2-CONH_2$, $-NH-CH_2CH_2-CO-NHCH_3$, $-N(CH_3)-CH_2CH_2-CONH_2$ oder $-N(CH_3)-CH_2CH_2-CO-NHCH_3$; -CO-alkyl-CO-NHZ wie $-CO-CH_2-CONH_2$, $-CO-CH_2-CO-NHCH_3$, $-CO-CH_2CH_2-CONH_2$ oder $-CO-CH_2CH_2-CO-NHCH_3$; -NZ-CO-alkyl-CO-NHZ wie $-NH-CO-CH_2CH_2-CONH_2$; -CO-NZ-alkyl-CO-NHZ wie $-CO-NH-CH_2-CONH_2$ oder $-CO-NH-CH_2-CO-NHCH_3$; -CO-NH-(2-pyridon-3-, -4-, -5- oder -6-yl); $-CH_2-CO-NH-$(2-pyridon-3-, -4-, -5- oder -6-yl); -NH-CO-NH-(2-pyridon-3-, -4-, -5- oder -6-yl); $-O-CH_2-CO-NH-$(2-pyridon-3-, -4-, -5- oder -6-yl); -CO-NH-(4-pyridon-2- oder -3-yl); $-CH_2-CO-NH-$(4-pyridon-2- oder -3-yl); -NH-CO-NH-(4-pyridon-2- oder -3-yl); $-CH_2-CO-NH-$(4-pyridon-2-oder-3-yl); 2-Oxo-imidazolidin-1-yl; 3-Methyl-2-oxo-imidazolidin-1-yl; 2-Oxo-imidazolidin-1-yl-carbonyl; 3-Methyl-2-oxo-imidazolidin-1-yl-carbonyl; 2-Oxo-imidazolidin-1-yl-carbonylmethyl; 3-Methyl-2-oxo-1-imidazolidin-1-yl-carbonylmethyl; 2-Oxo-imidazolidin-1-yl-carboxamido; 3-Methyl-2-oxo-imidazolidin-1-yl-carboxamido; 2-Oxo-imidazolidin-1-yl-carbonylmethoxy; 3-Methyl-2-oxo-imidazolidin-1-yl-carbonylmethoxy; $-SO_2-NHZ$ wie $SO_2NH_2$ oder $SO_2NHCH_3$; $-alkyl-SO_2-NHZ$ wie $-CH_2-SO_2NH_2$ oder $-CH_2CH_2SO_2NHCH_3$; $-O-alkyl-SO_2-NHZ$ wie $-O-CH_2-SO_2NH_2$; $-NZ-alkyl-SO_2-NHZ$ wie $-NH-CH_2-SO_2NH_2$, $-NH-CH_2-SO_2-NHCH_3$, $-N-(CH_3)-CH_2-SO_2NH_2$, $-N(CH_3)-CH_2-SO_2NHCH_3$, $-NH-CH_2CH_2-SO_2NH_2$, $-NH-CH_2CH_2-SO_2-NHCH_3$, $-N(CH_3)-CH_2CH_2-SO_2NH_2$ oder $-N(CH_3)-CH_2CH_2-SO_2NHCH_3$; $-CO-alkyl-SO_2NHZ$ wie$-CO-CH_2CH_2-SO_2NH_2$; $-NZ-CO-alkyl-SO_2NHZ$ wie $-NH-CO-CH_2CH_2-SO_2NH_2$; $-CO-NZ-alkyl-SO_2NHZ$ wie $-CO-NH-CH_2-SO_2NH_2$.

In den Verbindungen der Formel I trägt der Benzolring (außer $R^1$ und der dazu in o-Stellung stehenden, die Reste $R^2$, $R^3$ und $R^4$ tragenden Gruppe) vorzugsweise keine weiteren Substituenten. Falls der Benzolring jedoch zusätzlich substituiert ist, so ist er vorzugsweise einfach substituiert, und zwar bevorzugt in p-Stellung zum Rest $R^1$, also in 6-Stellung in den Isoindolinen Ia, Ib oder Ic, in 7-Stellung in den Tetrahydroisochinolinen Id, Ie oder If usw. Bevorzugte Substituenten sind dort $NO_2$, $NH_2$ oder die Gruppe

E-Q, die ihrerseits dort bevorzugt $-NH-CO-NH_2$, -N-CO-Z wie $-NH-CO-CH_3$, -NH-CO-alkyl-COOR$^5$ wie $-NH-CO-CH_2CH_2-COOC_2H_5$, -NH-CO-alkyl-CONHZ wie $-NH-CO-CH_2CH_2-CONH_2$ oder 2-Oxo-imidazolidin-1-yl bedeutet.

Besonders bevorzugte Reste $R^3$ sind 3,4-Dichlorphenyl, o-, m- oder p-Nitrophenyl, o-, m- oder p-Aminophenyl, o-, m- oder p-Ureidophenyl, o-, m- oder p-Carbamoylmethylphenyl, 1-Naphthyl und 4-Benzothienyl. Der Rest $R^3$ steht vorzugsweise aber auch für Phenyl, o-, m-oder p-Tolyl, o-, m- oder p-Ethylphenyl, o-, m- oder p-Methoxyphenyl, o-, m- oder p-Ethoxyphenyl, o-, m-oder p-Hydroxyphenyl, o-, m-oder p-Fluorphenyl, o-, m- oder p-Chlorphenyl, o-, m- oder p-Bromphenyl, o-, m- oder p-Carboxyphenyl, o-, m- oder p-Methoxycarbonylphenyl, o-, m- oder p-Ethoxycarbonylphenyl, o-, m-oder p-Methoxymethoxycarbonylphenyl, o-, m- oder p-Ethoxymethoxycarbonylphenyl, o-, m- oder p-Acetoxymethoxycarbonylphenyl, o-, m- oder p-(1-Acetoxyethoxycarbonyl)-phenyl, o-, m- oder p-Trimethylacetoxymethoxy carbonyl, o-, m-oder p-(1-Trimethylacetoxyethoxycarbonyl)-phenyl, o-, m- oder p-Methoxycarbonyloxymethoxycarbonylphenyl, o-, m- oder p-Ethoxycarbonyloxymethoxycarbonyl-phenyl, o-, m- oder p-(1-Methoxycarbonyloxyethoxycarbonyl)-phenyl, o-, m- oder p-(1-Ethoxycarbonyloxyethoxycarbonyl)-phenyl, o-, m- oder p-(2,3-Isopropylidendioxypropyl-oxycarbonyl)-phenyl, o-, m- oder p-Carboxymethylphenyl, o-, m- oder p-Methoxycarbonylmethylphenyl, o-, m- oder p-Ethoxycarbonylmethylphenyl, o-, m- oder p-Acetoxymethoxycarbonylmethylphenyl, o-, m- oder p-(1-Acetoxyethoxycarbonylmethyl)-phenyl, o-, m- oder p-Trimethylacetoxymethoxycarbonylmethylphenyl, o-, m-oder p-(1-Trimethylacetoxyethoxycarbonylmethyl)-phenyl, o-, m-oder p-Methoxycarbonyloxymethoxycarbonylmethylphenyl, o-, m- oder p-Ethoxycarbonyloxymethoxycarbonylmethylphenyl, o-, m- oder p-(1-Methoxycarbonyloxyethoxycarbonylmethyl)-phenyl, o-, m- oder p-(1-Ethoxycarbonyloxyethoxycarbonylmethyl)-phenyl, o-, m- oder p-(2,3-Isopropylidendioxypropyl-oxycarbonylmethyl)-phenyl, o-, m-oder p-Carboxymethoxyphenyl, o-, m- oder p-Methoxycarbonylmethoxyphenyl, o-, m-oder p-Ethoxycarbonylmethoxyphenyl, o-, m- oder p-Acetoxymethoxycarbonylmethoxyphenyl, o-, m- oder p-(1-Acetoxyethoxycarbonylmethoxy)-phenyl, o-, m- oder p-Trimethylacetoxymethoxycarbonylmethoxyphenyl, o-, m- oder p-(1-Trimethylacetoxyethoxycarbonylmethoxy)-phenyl, o-, m- oder p-Methoxycarbonyloxymethoxycarbonylmethoxyphenyl, o-, m- oder p-Ethoxycarbonyloxymethoxycarbonylmethoxyphenyl, o-, m-oder p-(1-Methoxycarbonyloxyethoxycarbonylmethoxy)-phenyl, o-, m- oder p-(1-Ethoxycarbonyloxyethoxycarbonylmethoxy)-phenyl, o-, m- oder p-(2,3-Isopropylidendioxypropyl-oxycarbonylmethoxy)-phenyl, 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Difluorphenyl, 2,3-, 2,4-, 2,5-, 2,6- oder 3,5-Dichlorphenyl, 2,3-, 2,4-, 2,5-, 2,6-, 3,4- oder 3,5-Dibromphenyl, 2-Chlor-4-hydroxyphenyl, 3-Chlor-4-hydroxyphenyl, 2-Hydroxy-4-chlorphenyl, 3-Hydroxy-4-chlorphenyl, 3-Chlor-4-carboxymethoxyphenyl, 3-Chlor-4-methoxycarbonylmethoxyphenyl, 3-Chlor-4-ethoxycarbonylmethoxyphenyl, 2- oder 3-Thienyl, 3-, 4-, oder 5-Chlor-2-thienyl, 3-, 4-, oder 5-Brom-2-thienyl, 2-, 4-, oder 5-Chlor-3-thienyl, 2-, 4-, oder 5-Brom-3-thienyl, 3,4-, 3,5- oder 4,5-Dichlor-2-thienyl, 2-Naphthyl, 2-, 3-, 4-, 5-, 6-, 7- oder 8-Chlor-1-naphthyl, 2-, 3-, 5-, 6- oder 7-Benzothienyl, o-, m- oder p-Carbamoylphenyl, o-, m- oder p-Carbamoylmethoxyphenyl, o-, m- oder p-Formamidophenyl, o-, m- oder p-Acetamidophenyl, o-, m-oder p-Methylsulfonamidophenyl, o-, m- oder p-N′-Methylureidophenyl, o-, m- oder p-N-Methylcarbamoylphenyl, o-, m- oder p-Aminooxalylaminophenyl ($H_2N-CO-CO-NH-C_6H_4$), 2-, 3- oder 4-Pyridyl.

$R^4$ ist vorzugsweise H, ferner bevorzugt E-Q, insbesondere $-NH-CO-NH_2$.

$R^5$ ist vorzugsweise H, A (insbesondere Methyl oder Ethyl), AO-alkyl (insbesondere Methoxymethyl, Ethoxymethyl, 1- oder 2-Methoxy-ethyl, 1- oder 2-Ethoxy-ethyl), A-CO-O-alkyl (insbesondere Acetoxymethyl, 1- oder 2-Acetoxyethyl, Trimethylacetoxymethyl, 1- oder 2-Trimethylacetoxyethyl) oder AO-CO-O-alkyl (insbesondere Methoxycarbonyloxymethyl, Ethoxycarbonyloxymethyl, 1- oder 2-Methoxycarbonyloxy-ethyl, 1- oder 2-Ethoxycarbonyloxyethyl).

Bevorzugt sind auch Verbindungen, in denen $R^5$ eine $R^6OCH_2-CH(OR^7)-CH_2$-Gruppe bedeutet. Darin stehen $R^6$ und $R^7$ jeweils für A (bevorzugt für Methyl oder Ethyl) oder zusammen für Alkyliden oder Cycloalkyliden mit jeweils bis zu 6 C-Atomen (bevorzugt für Isopropyliden, ferner bevorzugt für 2-Butyliden, Cyclopentyliden, Cyclohexyliden).

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die Teilformeln I′ sowie Ia′ bis Io′ ausgedrückt werden, die den Formeln I (sowie Ia bis Io) entsprechen und worin die nicht näher bezeichneten Reste die bei der Formel I angegebenen Bedeutungen haben, worin jedoch $R^3$ Phenyl, Tolyl, Methoxyphenyl, Hydroxyphenyl, Fluorphenyl, Chlorphenyl, Bromphenyl, Carboxyphenyl, Methoxycarbonylphenyl, Ethoxycarbonylphenyl, Carboxymethylphenyl, Methoxycarbonylmethylphenyl, Ethoxycarbonylmethylphenyl, Carboxymethoxyphenyl, Methoxycarbonylmethoxyphenyl, Ethoxycarbonylmethoxyphenyl, Dichlorphenyl, Chlorhydroxyphenyl, Thienyl, Naphthyl, Benzothienyl, Nitrophenyl, Aminophenyl, Ureidophenyl,

Carbamoylmethylphenyl, Carbamoylphenyl, Carbamoylmethoxyphenyl, Formamidophenyl, Acetamidophenyl, Methylsulfonamidophenyl, N′-Methylureidophenyl, N-Methylcarbamoylphenyl, Aminooxalylaminophenyl oder 2-, 3- oder 4-Pyridyl bedeutet.

Ferner sind bevorzugt Verbindungen der Formeln I″ sowie Ia″ bis Io″, die den Formeln I sowie Ia bis Io entsprechen, worin jedoch

$R^3$ Phenyl, Tolyl, Methoxyphenyl, Chlorphenyl, Bromphenyl, Dichlorphenyl, Chlorhydroxyphenyl, Thienyl, Naphthyl, Benzothienyl, Nitrophenyl, Aminophenyl, Ureidophenyl oder Carbamoylmethylphenyl bedeutet.

Weiterhin sind bevorzugt Verbindungen der Formeln I‴ sowie Ia‴ bis Io‴, die den Formeln I sowie Ia bis Io entsprechen, worin jedoch

$R^3$ 3,4-Dichlorphenyl, 1-Naphthyl, 4-Benzothienyl, o-, m- oder p-Ureidophenyl oder o-, m- oder p-Carbamoylmethylphenyl

bedeutet.

Insbesondere sind diejenigen Verbindungen der Formeln I, Ia bis Io, I′, Ia′ bis Io′, I″, Ia″ bis Io″, I‴ sowie Ia‴ bis Io‴ bevorzugt, in denen der Benzolring keine zusätzlichen Substituenten trägt.

Weiterhin sind diejenigen unter allen genannten Verbindungen bevorzugt, in denen $R^4$ H bedeutet.

Gegenstand der Erfindung ist ferner ein Verfahren zur Herstellung von stickstoffhaltigen Ringverbindungen der Formel I sowie ihrer Salze, dadurch gekennzeichnet, daß man eine Verbindung der Formel II

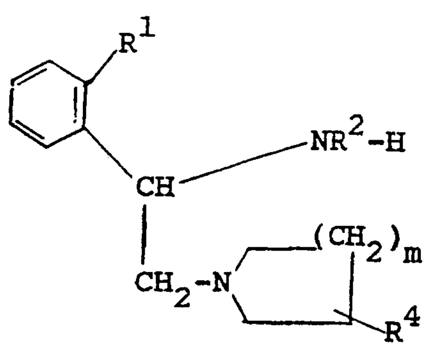

II

worin im Benzolring eine CH-Gruppe durch N ersetzt und/oder der Benzolring wie angegeben substituiert sein kann und $R^1$, $R^2$, $R^4$ und m die angegebenen Bedeutungen haben,

mit einer Verbindung der Formel III

$HOOC\text{-}CH_2\text{-}R^3$ III

worin $R^3$ die angegebene Bedeutung hat und wie angegeben substituiert sein kann,

oder einem ihrer funktionellen Derivate umsetzt

oder daß man eine Verbindung der Formel IV

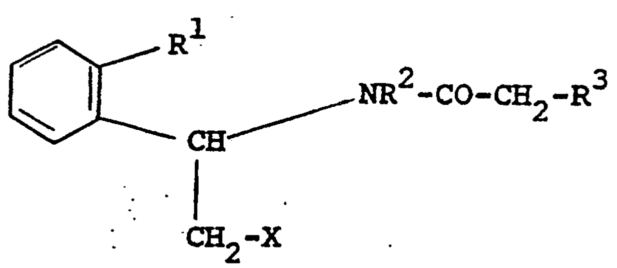

IV

worin im Benzolring und/oder im Rest $R^3$ unabhängig voneinander jeweils eine CH-Gruppe durch N ersetzt sein und/oder der Benzolring und/oder der Rest $R^3$ unabhängig voneinander jeweils wie angegeben substituiert sein kann (können),

X $X^1$ oder $NH_2$ und

$X^1$ Cl, Br, J oder eine freie oder funktionell abgewandelte OH-Gruppe bedeuten und

$R^1$, $R^2$ und $R^3$ die angegebenen Bedeutungen haben,

mit einer Verbindung der Formel V

$X^2\text{-}CH_2\text{-}(CH_2)_m\text{-}CH_2\text{-}CH_2\text{-}X^3$ V

worin
ein Wasserstoffatom in einer der $CH_2$-Gruppen durch den Rest $R^4$ ersetzt ist,
$X^2$ und $X^3$ zusammen NH oder, falls X $NH_2$ bedeutet, jeweils $X^1$ bedeuten und
m die angegebene Bedeutung hat,
umsetzt
oder daß man eine Verbindung, die sonst der Formel I entspricht, aber an Stelle eines oder mehrerer H-Atome eine oder mehrere reduzierbare oder hydrogenolytisch abspaltbare Gruppen und/oder C-C- und/oder C-N-Bindungen enthält, mit einem reduzierenden Mittel behandelt,
und/oder daß man in einer Verbindung der Formel I einen oder mehrere der Reste $R^3$, $R^4$, E und/oder Q gegen einen oder mehrere andere Reste $R^3$, $R^4$, E und/oder Q austauscht,
und/oder daß man eine Base bzw. Säure der Formel I durch Behandeln mit einer Säure bzw. Base in eines ihrer Salze überführt.

Die Verbindungen der Formel I werden in der Regel nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind in der Regel bekannt, oder sie können in Analogie zu bekannten Stoffen nach an sich bekannten Verfahren hergestellt werden. Sie können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die einzelnen Verfahrensvarianten werden im folgenden näher erläutert.

Die Verbindungen der Formel I sind bevorzugt herstellbar durch Reaktion der Verbindungen der Formel II mit Carbonsäuren der Formel III oder ihren reaktionsfähigen funktionellen Derivaten. Als reaktionsfähige funktionelle Derivate der Verbindungen der Formel III eignen sich insbesondere die entsprechenden Ester, vor allem die Methyl-oder Ethylester, die Halogenide, Anhydride, Azide oder Nitrile.

Verbindungen der Formel II sind beispielsweise erhältlich durch Reduktion von Verbindungen der Formel VI

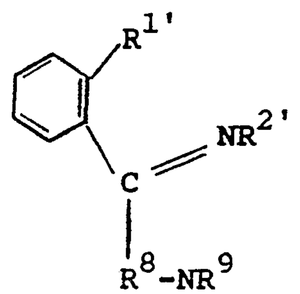

worin im Benzolring eine CH-Gruppe durch N ersetzt und/oder der Benzolring wie angegeben substituiert sein kann,
$R^{1'}$ und $R^{2'}$
(a) die für $R^1$ bzw. $R^2$ angegebenen Bedeutungen haben
(b) zusammen $-CH=CH-$, $-CH_2-CH=CH-$ oder $-CH=CH-CH_2-$, bedeuten,
$R^8$
(a) $-CH_2-$,
(b) -CO- und
$R^9$
(a) eine $-(CH_2)_{m+3}$-Kette, worin ein H-Atom durch den Rest $R^4$ ersetzt ist
(b) eine $-(CH_2)_{m+3}$-Kette, worin ein H-Atom durch den Rest $R^4$ ersetzt ist, die aber mindestens eine C-C-Doppelbindung enthält, bedeuten und
m die angegebenen Bedeutungen hat, worin jedoch nicht gleichzeitig alle Reste $R^{1'}$, $R^{2'}$, $R^8$ und $R^9$ die unter (a) jeweils angegebenen Bedeutungen haben können.

Typische Verbindungen der Formel II sind z.B. 1-Pyrrolidinomethyl-, 1-Piperidinomethyl- und 1-Hexahydroazepinomethyl-isoindolin, 1-Pyrrolidinomethyl-, 1-Piperidinomethyl- und 1-Hexahydroazepinomethyl-1,2,3,4-tetrahydroisochinolin, 1-Pyrrolidinomethyl-, 1-Piperidinomethyl-und 1-Hexahydroazepinomethyl-2,3,4,5-tetrahydro-1H-2-benzazepin, 2-Methylamino-2-phenyl-ethyl-pyrrolidin, 2-, 3- oder 4-(1-Methylamino-2-pyrrolidino-ethyl)pyridin.

Typische Verbindungen der Formel III sind z.B. Phenylacetylchlorid, -bromid und -azid, Phenylessigsäuremethyl- und -ethylester, (Phenylessigsäure)-anhydrid, Phenylacetonitril sowie die entsprechenden Derivate der 3,4-Dichlorphenylessigsäure (z.B. 3,4-Dichlorphenylacetylchlorid), der 1-Naphthylessigsäure (z.B. 1-Naphthylacetylchlorid), der 4-Benzothienylessigsäure (z.B. 4-Benzothienylacetylchlorid) und der o-, m- oder p-Nitrophenylessigsäure (z.B. o-, m- oder p-Nitrophenylacetylchlorid).

Die Umsetzung von II mit III bzw. III-Derivaten gelingt zweckmäßig in Anwesenheit oder Abwesenheit eines inerten organischen Lösungsmittels, z.B. eines halogenierten Kohlenwasserstoffs wie Dichlormethan, Chloroform oder Trichlorethen, eines Alkohols wie Methanol, Ethanol oder Butanol, eines Ethers wie Tetrahydrofuran (THF) oder Dioxan, eines Amids wie Dimethylformamid (DMF), eines Sulfoxids wie Dimethylsulfoxid (DMSO) und/oder in Gegenwart oder Abwesenheit eines Kondensationsmittels, z.B. einer Base, bei Temperaturen zwischen, -20 und 200°, vorzugsweise 0 und 100°. Als Basen eignen sich z.B. Alkalimetallhydroxide wie NaOH oder KOH, Alkalimetallcarbonate wie $Na_2CO_3$ oder $K_2CO_3$, tertiäre Amine wie Triethylamin oder Pyridin. Als Lösungsmittel ist Dichlormethan, als Base Triethylamin besonders bevorzugt.

Eine Reaktion der Verbindungen der Formeln IV und V führt ebenfalls zu Verbindungen der Formel I. Diese Umsetzung wird zweckmäßig in einem der angegebenen inerten Lösungsmittel bei Temperaturen zwischen etwa 0 und etwa 200°, vorzugsweise zwischen 10 und 120° vorgenommen, wobei der Zusatz einer Base vorteilhaft sein kann.

Verbindungen der Formel IV (X = OH) sind z.B. erhältlich durch Acylierung entsprechender 1-Hydroxymethyl-isoindoline, -1,2,3,4-tetrahydroisochinoline oder -2,3,4,5-tetrahydro-1H-2-benzazepine bzw. entsprechende 2-$R^2$-NH-2-phenylethanole oder 2-$R^2$NH-2-(pyridyl)-ethanole mit Verbindungen der Formel III oder ihren Derivaten. Die übrigen Verbindungen der Formel IV sind daraus in üblicher Weise herstellbar, IV (X = Br) z.B. mit HBr in Essigsäure, IV (X = $NH_2$) aus der Bromverbindung mit Phthalimid-Kalium und nachfolgende Hydrolyse. Verbindungen der Formel IV (X = $NH_2$) sind auch herstellbar durch Reduktion von Reissert-Verbindungen (entsprechend Formel IV, aber CN an Stelle von $CH_2$-X).

Verbindungen der Formel V ($X^2$ = $X^3$ = OH) sind z.B. erhältlich durch Reduktion von Estern der Formel AOOC-$(CH_2)_m$-$CH_2$-COOA. Die übrigen Verbindungen der Formel V sind daraus in üblicher Weise herstellbar, V ($X^2$ = $X^3$ = Br) z.B. mit $SOBr_2$.

Als Ausgangsstoffe für die Herstellung von Verbindungen der Formel I durch Reduktion entsprechender Verbindungen, die an Stelle von H-Atomen eine oder mehrere zusätzliche reduzierbare oder hydrogenolytisch abspaltbare Gruppen und/oder C-C-Bindungen und/oder C-N-Bindungen enthalten, eignen sich insbesondere Verbindungen der Formel VII

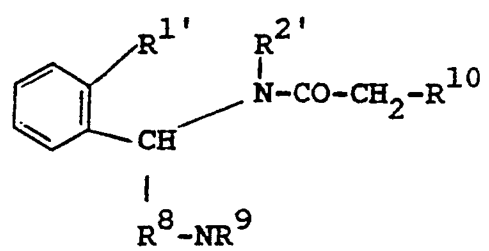

VII

worin

$R^{10}$

(a) $R^3$,

(b) einen sonst $R^3$ entsprechenden Rest, der aber an Stelle des H-Atoms einer OH-Gruppe einen hydrogenolytisch abspaltbaren Rest enthält, bedeutet und

$R^{1'}$, $R^{2'}$, $R^3$, $R^8$ und $R^9$, die angegebenen Bedeutungen haben;

dabei muß VII jedoch von I verschieden sein, d.h. die Reste $R^{1'}$, $R^{2'}$, $R^8$, $R^9$ und $R^{10}$ können nicht gleichzeitig die jeweils unter (a) angegebenen Bedeutungen haben.

Der Rest $R^{10}$ kann vorzugsweise o-, m- oder p-Benzyloxyphenylacetyl bedeuten.

Als reduzierendes Mittel eignet sich vorzugsweise Wasserstoff in Gegenwart eines Katalysators, insbesondere eines Edelmetall-, Nickel- oder Kobaltkatalysators. Als Edelmetalle kommen in erster Linie Platin und Palladium in Betracht, die auf Trägern (z.B. auf Kohle, Calciumcarbonat oder Strontiumcarbonat), als Oxide (z.B. Platinoxid) oder in feinteiliger Form vorliegen können. Nickel- und Kobaltkatalysatoren

werden zweckmäßig als Raney-Metalle eingesetzt. Man hydriert zweckmäßig bei Drucken zwischen etwa 1 und etwa 200 bar und bei Temperaturen zwischen etwa -80 und +150°, vorzugsweise zwischen 20 und 100°, in Gegenwart eines inerten Lösungsmittels, z.B. eines Alkohols wie Methanol, Ethanol oder Isopropanol, einer Carbonsäure wie Essigsäure, eines Esters wie Ethylacetat, eines Ethers wie THF oder Dioxan.

Falls erwünscht, kann man in einer Verbindung der Formel I einen oder mehrere der Reste $R^3$, $R^4$, E und/oder Q gegen einen oder mehrere andere Reste $R^3$, $R^4$, E und/oder Q austauschen.

So kann man Ethergruppen (z.B. OA-Gruppen) unter Bildung von OH-Gruppen spalten, z.B. durch Behandeln mit Dimethylsulfid-Bortribromid-Komplex, z.B. in Toluol, THF oder DMSO, oder durch Verschmelzen mit Pyridin- oder Anilin hydrohalogeniden, vorzugsweise Pyridinhydrochlorid, bei etwa 150-250°, oder durch Behandeln mit Diisobutylaluminiumhydrid in Toluol bei etwa 0-110°.

Ferner kann man Estergruppen (z.B. COOA-Gruppen) unter Bildung von COOH-Gruppen hydrolysieren, zweckmäßig mit NaOH oder KOH in Alkoholen wie Methanol oder Ethanol oder deren Gemischen mit Wasser bei Temperaturen zwischen 0 und 100°.

Weiterhin kann man OH-Gruppen verethern oder verestern, z.B. indem man zunächst die entsprechenden Alkalimetall-(z.B. Na- oder K-)alkoholate, -phenolate oder Salze herstellt und diese mit entsprechenden Halogenverbindungen umsetzt, z.B. mit Alkylhalogeniden wie Methylchlorid, -bromid oder -iodid, Chlor- oder Bromessigsäure-methyl oder -ethylester, Methoxymethylchlorid, 2,3-Isopropylidendioxypropylchlorid, zweckmäßig in Gegenwart eines der oben angegebenen Lösungsmittel bei Temperaturen zwischen 0 und 100°.

Nitrogruppen können zu Aminogruppen reduziert werden, zweckmäßig durch katalytische Hydrierung unter den oben angegebenen Bedingungen, z.B. mit Raney-Ni im Methanol oder Ethanol bei 15-40° und Normaldruck.

Aminogruppen können acyliert werden, z.B. mit Säurechloriden wie Acetyl-, Methansulfonyl-, Oxalsäure- oder Bernsteinsäurehalbester-chlorid, zweckmäßig in inerten Lösungsmitteln wie Dichlormethan bei 15-40°. Eine Formylierung von Aminogruppen gelingt auch durch mehrstündige Reaktion mit überschüssiger Ameisensäure bei 80-100°. Umsetzung von primären Aminoverbindungen mit Cyanaten, z.B. mit KCNO in Wasser bei 15-40°, gibt die entsprechenden Ureidoverbindungen; mit Alkylisocyanaten, z.B. in inerten Lösungsmitteln wie THF bei 15-40°, erhält man entsprechend N′-Alkyl-ureidoverbindungen.

Carbonsäuren können in Amide übergeführt werden, zweckmäßig durch Reaktion mit Ammoniak oder Alkylaminen in Gegenwart eines Dehydratisierungsmittels wie Dicyclohexylcarbodiimid oder Carbonyldiimidazol in einem inerten Lösungsmittel wie DMF bei 15-40°.

Eine Base der Formel I kann mit einer Säure in das zugehörige Säureadditionssalz übergeführt werden. Für diese Umsetzung kommen Säuren in Frage, die physiologisch unbedenkliche Salze liefern. So können anorganische Säuren verwendet werden, z .B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Aufreinigung der Verbindungen der Formel I verwendet werden.

Eine Säure der Formel I (z.B. $R^3$ = COOH) kann umgekehrt durch Behandeln mit einer Base in eines ihrer Metall- bzw. Ammoniumsalze übergeführt werden. Als Salze kommen insbesondere die Natrium-, Kalium-, Magnesium-, Calcium- und Ammoniumsalze in Betracht, ferner substituierte Ammoniumsalze.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I enthalten mindestens ein chirales Zentrum und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z. B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie ß-Camphersulfonsäure. Vorteilhaft ist auch eine Enantiomerentrennung mit Hilfe einer mit einem optisch aktiven Trennmittel (z.B. Dinitrobenzoyl-phenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch-aktive Verbindungen der Formel I nach den oben beschriebenen

Methoden zu erhalten, indem man Ausgangsstoffe (z.B. solche der Formel II) verwendet, die bereits optisch-aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks-und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Schmerzzuständen verwendet werden.

Dabei werden die erfindungsgemäßen Substanzen in der Regel in Analogie zu bekannten Analgetika verabreicht, vorzugsweise in Dosierungen zwischen etwa 1 und 500 mg, insbesondere zwischen 5 und 100 mg pro Dosierungseinheit. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser oder verdünnte Natronlauge hinzu, extrahiert mit Dichlormethan trennt ab, trocknet die organische Phase mit Natriumsulfat, filtriert, dampft ein und reinigt durch Chromatographie an Kieselgel und/oder durch Kristallisation.

HCl′ = Hydrochlorid. Rf = Rf-Wert auf Dünnschicht-Kieselgel 60 $F_{254}$ (E. Merck, Art.-Nr. 5715), $CH_2Cl_2/CH_3OH$ 9:1 mit 0,1 % Triethylamin.

$[\alpha] = [\alpha]_D^{20}$, c = 1 in Methanol.

Beispiel 1

Eine Lösung von 21,6 g 1-Pyrrolidinomethyl-1,2,3,4-tetrahydroisochinolin (erhältlich durch Reduktion von 1-Pyrrolidinomethylisochinolin mit Na in Ethanol) in 250 ml Dichlormethan wird mit 30 g Triethylamin versetzt. Anschließend tropft man unter Rühren eine Lösung von 22,4 g 3,4-Dichlorphenylacetylchlorid in 250 ml Dichlor methan hinzu, rührt noch 2 Std. bei 20° und erhält nach üblicher Aufarbeitung 1-Pyrrolidinomethyl-2-(3,4-dichlorphenylacetyl)-1,2,3,4-tetrahydroisochinolin ("P"). HCl′, F. 264°.

Verwendet man als Ausgangsstoff die enantiomeren 1-Pyrrolidinomethyl-1,2,3,4-tetrahydroisochinoline, so erhält man analog die beiden enantiomeren Formen des 1-Pyrrolidinomethyl-2-(3,4-dichlorphenylacetyl)-1,2,3,4-tetrahydroisochinolins.

Analog erhält man die nachstehenden 1,2,3,4-Tetrahydroisochinoline:

1-Pyrrolidinomethyl-2-phenylacetyl-, HCl′, F. 277°
1-Pyrrolidinomethyl-2-o-tolylacetyl-
1-Pyrrolidinomethyl-2-m-tolylacetyl-, HCl′, F. 242°
1-Pyrrolidinomethyl-2-p-tolylacetyl-
1-Pyrrolidinomethyl-2-o-methoxyphenylacetyl-

1-Pyrrolidinomethyl-2-m-methoxyphenylacetyl-, HCl′, F. 219°
1-Pyrrolidinomethyl-2-p-methoxyphenylacetyl-
1-Pyrrolidinomethyl-2-o-ethoxyphenylacetyl-
1-Pyrrolidinomethyl-2-m-ethoxyphenylacetyl-
1-Pyrrolidinomethyl-2-p-ethoxyphenylacetyl-
1-Pyrrolidinomethyl-2-o-fluorphenylacetyl-
1-Pyrrolidinomethyl-2-m-fluorphenylacetyl-
1-Pyrrolidinomethyl-2-p-fluorphenylacetyl-
1-Pyrrolidinomethyl-2-o-chlorphenylacetyl-
1-Pyrrolidinomethyl-2-m-chlorphenylacetyl-, HCl′, F. 219°
1-Pyrrolidinomethyl-2-p-chlorphenylacetyl-, HCl′, F. 259°
1-Pyrrolidinomethyl-2-o-bromphenylacetyl-
1-Pyrrolidinomethyl-2-m-bromphenylacetyl-
1-Pyrrolidinomethyl-2-p-bromphenylacetyl-, HCl′, F. 261°
1-Pyrrolidinomethyl-2-o-methoxycarbonylphenylacetyl-
1-Pyrrolidinomethyl-2-m-methoxycarbonylphenylacetyl-
1-Pyrrolidinomethyl-2-p-methoxycarbonylphenylacetyl-, HCl′, F. 233°
1-Pyrrolidinomethyl-2-o-ethoxycarbonylphenylacetyl-
1-Pyrrolidinomethyl-2-m-ethoxycarbonylphenylacetyl-
1-Pyrrolidinomethyl-2-p-ethoxycarbonylphenylacetyl-
1-Pyrrolidinomethyl-2-o-methoxycarbonylmethyl-phenylacetyl-, HCl′, F. 199°
1-Pyrrolidinomethyl-2-m-methoxycarbonylmethyl-phenylacetyl-, HCl′, F. 239°
1-Pyrrolidinomethyl-2-p-methoxycarbonylmethyl-phenylacetyl-
1-Pyrrolidinomethyl-2-o-ethoxycarbonylmethyl-phenylacetyl-
1-Pyrrolidinomethyl-2-m-ethoxycarbonylmethyl-phenylacetyl-
1-Pyrrolidinomethyl-2-p-ethoxycarbonylmethyl-phenylacetyl-
1-Pyrrolidinomethyl-2-o-methoxycarbonylmethoxy-phenylacetyl-
1-Pyrrolidinomethyl-2-m-methoxycarbonylmethoxy-phenylacetyl-, HCl′, F. 188°
1-Pyrrolidinomethyl-2-p-methoxycarbonylmethoxy-phenylacetyl-
1-Pyrrolidinomethyl-2-o-ethoxycarbonylmethoxy-phenylacetyl-
1-Pyrrolidinomethyl-2-m-ethoxycarbonylmethoxy-phenylacetyl-
1-Pyrrolidinomethyl-2-p-ethoxycarbonylmethoxy-phenylacetyl-
1-Pyrrolidinomethyl-2-(3,4-dimethoxyphenylacetyl)-, HCl′, F. 232°
1-Pyrrolidinomethyl-2-(3,4-difluorphenylacetyl)-
1-Pyrrolidinomethyl-2-(2,3-dichlorphenylacetyl)-
1-Pyrrolidinomethyl-2-(2,4-dichlorphenylacetyl)-
1-Pyrrolidinomethyl-2-(2,5-dichlorphenylacetyl)-
1-Pyrrolidinomethyl-2-(2,6-dichlorphenylacetyl)-
1-Pyrrolidinomethyl-2-(3,5-dichlorphenylacetyl)-
1-Pyrrolidinomethyl-2-(3,4-dibromphenylacetyl)-
1-Pyrrolidinomethyl-2-(3-chlor-4-methoxycarbonylmethoxyphenylacetyl)-
1-Pyrrolidinomethyl-2-(3-chlor-4-ethoxycarbonylmethoxyphenylacetyl)-
1-Pyrrolidinomethyl-2-(2-thienylacetyl)-, HCl′, F. 275°
1-Pyrrolidinomethyl-2-(1-naphthylacetyl)-, HCl′, F. 262°
1-Pyrrolidinomethyl-2-(2-naphthylacetyl)-, HCl′, F. 255°
1-Pyrrolidinomethyl-2-(2-benzothienyl-acetyl)-
1-pyrrolidinomethyl-2-(3-benzothienyl-acetyl)-, HCl′, F. 208°
1-Pyrrolidinomethyl-2-(4-benzothienyl-acetyl)-, HCl′, F. 269°
1-Pyrrolidinomethyl-2-(5-benzothienyl-acetyl)-
1-Pyrrolidinomethyl-2-(6-benzothienyl-acetyl)-
1-Pyrrolidinomethyl-2-(7-benzothienyl-acetyl)-
1-Pyrrolidinomethyl-2-(3,4-dichlorphenylacetyl)-6,7-dimethoxy-
1-Pyrrolidinomethyl-2-(1-naphthylacetyl)-6,7-dimethoxy-
1-Pyrrolidinomethyl-2-(4-benzothienyl-acetyl)-6,7-dimethoxy-
1-Pyrrolidinomethyl-2-(3,4-dichlorphenylacetyl)-6,7-methylendioxy-
1-Pyrrolidinomethyl-2-(1-naphthylacetyl)-6,7-methylendioxy-
1-Pyrrolidinomethyl-2-(4-benzothienyl-acetyl)-6,7-methylendioxy-
1-Piperidinomethyl-2-(3,4-dichlorphenylacetyl)-, HCl′-hemihydrathemiacetonsolvat, F. 102°

1-Piperidinomethyl-2-(1-naphthylacetyl)-, HCl', F. 288°
1-Piperidinomethyl-2-(4-benzothienyl-acetyl)-, HCl', F. 278°
1-Hexahydroazepinomethyl-2-(3,4-dichlorphenylacetyl)-, HCl', F. 184°
1-Hexahydroazepinomethyl-2-(1-naphthylacetyl)-, HCl, F. 274°
1-Hexahydroazepinomethyl-2-(4-benzothienyl-acetyl)-.

Beispiel 2

Analog Beispiel 1 erhält man mit 1-Pyrrolidinomethylisoindolin das 1-Pyrrolidinomethyl-2-(3,4-dichlorphenylacetyl)-isoindolin.

Analog erhält man folgende Isoindoline:
1-Pyrrolidinomethyl-2-(1-naphthylacetyl)-
1-Pyrrolidinomethyl-2-(4-benzothienylacetyl)-
1-Piperidinomethyl-2-(3,4-dichlorphenylacetyl)-
1-Piperidinomethyl-2-(1-naphthylacetyl)-
1-Piperidinomethyl-2-(4-benzothienylacetyl)-
1-Hexahydroazepinomethyl-2-(3,4-dichlorphenylacetyl)-
1-Hexahydroazepinomethyl-2-(1-naphthylacetyl)-
1-Hexahydroazepinomethyl-2-(4-benzothienylacetyl)-
sowie folgende 2,3,4,5-Tetrahydro-1H-2-benzazepine:
1-Pyrrolidinomethyl-2-(3,4-dichlorphenylacetyl)-
1-Pyrrolidinomethyl-2-(1-naphthylacetyl)-
1-Pyrrolidinomethyl-2-(4-benzothienylacetyl)-
1-Piperidinomethyl-2-(3,4-dichlorphenylacetyl)-
1-Piperidinomethyl-2-(1-naphthylacetyl)-
1-Piperidinomethyl-2-(4-benzothienylacetyl)-
1-Hexahydroazepinomethyl-2-(3,4-dichlorphenylacetyl)-
1-Hexahydroazepinomethyl-2-(1-naphthylacetyl)-
1-Hexahydroazepinomethyl-2-(4-benzothienylacetyl)-.

Beispiel 3

Man löst 6 g 3-Chlor-4-hydroxyphenylessigsäurehydrazid (erhältlich aus dem Ester mit Hydrazinhydrat) in 200 ml Wasser und 40 ml 1 n Salzsäure, tropft unter Rühren bei 0-3° eine Lösung von 2,4 g $NaNO_2$ in 40 ml Wasser hinzu, rührt noch 30 Min. und extrahiert das gebildete Azid mit Dichlormethan. Nach Trocknen über Mg $SO_4$ und Konzentration auf 50 ml wird die Lösung unter Rühren zugetropft zu einer Lösung von 9 g 1-Pyrrolidinomethyl-1,2,3,4-tetrahydroisochinolin und 4,4 ml Triethylamin in 100 ml Dichlormethan. Man rührt noch 2 Std. bei 20°, arbeitet wie üblich auf und erhält 1-Pyrrolidinomethyl-2-(3-chlor-4-hydroxyphenylacetyl )-1,2,3,4-tetrahydroisochinolin. HCl', F. 178°.

Analog erhält man die nachstehenden 1,2,3,4-Tetrahydroisochinoline:
1-Pyrrolidinomethyl-2-o-hydroxyphenylacetyl-
1-Pyrrolidinomethyl-2-m-hydroxyphenylacetyl-
1-Pyrrolidinomethyl-2-p-hydroxyphenylacetyl-.

Beispiel 4

Eine Lösung von 41,3 g 1-Brommethyl-2-(3,4-dichlorphenylacetyl)-1,2,3,4-tetrahydroisochinolin (erhältlich aus 1-Brommethyl-1,2,3,4-tetrahydroisochinolin und 3,4-Dichlorphenylacetylchlorid) und 22 g Pyrrolidin in 600 ml THF 6 Std. gekocht, eingedampft und wie üblich aufgearbeitet. Man erhält "P", HCl', F. 264°.

Beispiel 5

Ein Gemisch von 34,9 g 1-Aminomethyl-2-(3,4-dichlorphenylacetyl)-1,2,3,4-tetrahydroisochinolin, 21,6 g

1,4-Dibrombutan, 15 g $K_2CO_3$ und 800 ml Ethanol wird 8 Std. gekocht, eingedampft und wie üblich aufgearbeitet. Man erhält "P"; HCl′, F. 264°.

Beispiel 6

Man hydriert eine Lösung von 1 g 1-Pyrrolidinomethyl-2-(1-naphthylacetyl)-1,2-dihydroisochinolin [erhältlich durch Umsetzung von Isochinolin mit 1-Naphthylacetylchlorid/KCN zu 1-Cyan-2-(1-naphthylacetyl)-1,2-dihydroisochinolin, Reduktion zur 1-Aminomethyl-verbindung und Reaktion mit 1,4-Dibrombutan] in 20 ml THF an 0,5 g 5%igem Pd-C bei 20° und 1 bar bis zur Aufnahme von 1 Äquivalent $H_2$, filtriert, dampft ein, arbeitet wie üblich auf und erhält 1-Pyrrolidinomethyl-2-(1-naphthylacetyl )-1,2,3,4-tetrahydroisochinolin, HCl′, F. 262°.

Beispiel 7

Man hydriert eine Lösung von 1 g 1-Piperidinomethyl-2-p-Benzyloxyphenylacetyl-1,2,3,4-tetrahydroisochinolin in 25 ml Ethylacetat an 0,5 g 5%ig. Pd-C bei 20° und 1 bar bis zum Stillstand der Wasserstoffaufnahme, filtriert, dampft ein und erhält 1-Piperidinomethyl-2-p-hydroxyphenylacetyl-1,2,3,4-tetrahydroisochinolin.

Beispiel 8

Ein Gemisch von 3,64 g 1-Pyrrolidinomethyl-2-m-methoxyphenylacetyl-1,2,3,4-tetrahydroisochinolin und 3,5 g Pyridinhydrochlorid wird 3 Std. auf 160° erhitzt, abgekühlt und wie üblich aufgearbeitet. Man erhält 1-Pyrrolidinomethyl-2-m-hydroxyphenylacetyl-1,2,3,4-tetrahydroisochinolin.

Beispiel 9

Man löst 2,3 g Na in 600 ml Methanol, gibt 38,5 g 1-Pyrrolidinomethyl-2-(3-chlor-4-hydroxyphenylacetyl)-1,2,3,4-tetrahydroisochinolin hinzu, rührt 2 Std. bei 20° und versetzt tropfenweise mit 10,9 g Chloressigsäuremethylester. Nach 1 Std. Kochen kühlt man ab, arbeitet wie üblich auf und erhält 1-Pyrrolidinomethyl-2-(3-chlor-4-methoxycarbonylmethoxy-phenylacetyl)-1,2,3,4-tetrahydroisochinolin. HCl′, F. 213°.

Beispiel 10

Ein Gemisch von 10 g 1-Pyrrolidinomethyl-2-(3-chlor-4-methoxycarbonylmethoxy-phenylacetyl)-1,2,3,4-tetrahydroisochinolin, 2 g KOH und 150 ml Ethanol wird 2 Std. gekocht. Man dampft ein, nimmt in Wasser aus, versetzt mit der berechneten Menge verdünnter Salzsäure und filtriert das ausgefallene 1-Pyrrolidinomethyl-2-(3-chlor-4-carboxymethoxy-phenylacetyl)-1,2,3,4-tetrahydroisochinolin ab; Dihydrat, F. 136°.

Analog erhält man durch Verseifung der entsprechenden Ester die nachstehenden 1,2,3,4-Tetrahydroisochinoline:

1-Pyrrolidinomethyl-2-o-carboxyphenylacetyl-
1-Pyrrolidinomethyl-2-m-carboxyphenylacetyl-, HCl′, Monohydrat, F. 154°
1-Pyrrolidinomethyl-2-p-carboxyphenylacetyl-, HCl′, Dihydrat, F. 167°
1-Pyrrolidinomethyl-2-o-carboxymethyl-phenylacetyl-
1-Pyrrolidinomethyl-2-m-carboxymethyl-phenylacetyl-
1-Pyrrolidinomethyl-2-p-carboxymethyl-phenylacetyl-
1-Pyrrolidinomethyl-2-o-carboxymethoxy-phenylacetyl-
1-Pyrrolidinomethyl-2-m-carboxymethoxy-phenylacetyl-, HCl′, F. > 240°
1-Pyrrolidinomethyl-2-p-carboxymethoxy-phenylacetyl-.

Beispiel 11

Ein Gemisch aus 46,5 g Na-Salz des 1-Pyrrolidinomethyl-2-(3-chlor-4-carboxymethoxy-phenylacetyl)-1,2,3,4-tetrahydroisochinolins, 15,1 g Trimethylacetoxymethylchlorid und 600 ml DMF wird 3 Std. bei 20° gerührt. Man dampft ein, arbeitet wie üblich auf und erhält 1-Pyrrolidinomethyl-2-(3-chlor-4-trimethylacetoxymethoxycarbonylmethoxy-phenylacetyl)-1,2,3,4-tetrahydroisochinolin.

Analog erhält man die nachstehenden 1,2,3,4-Tetrahydroisochinoline:
1-Pyrrolidinomethyl-2-(3-chlor-4-methoxymethoxycarbonylmethoxy-phenylacetyl)-
1-Pyrrolidinomethyl-2-(3-chlor-4-acetoxymethoxycarbonylmethoxy-phenylacetyl)-
1-Pyrrolidinomethyl-2-[3-chlor-4-(1-acetoxy-ethoxycarbonylmethoxy)-phenylacetyl]-
1-Pyrrolidinomethyl-2-[3-chlor-4-(1-trimethylacetoxyethoxycarbonylmethoxy)-phenylacetyl]-
1-Pyrrolidinomethyl-2-(3-chlor-4-methoxycarbonyloxymethoxycarbonylmethoxy-phenylacetyl)-
1-Pyrrolidinomethyl-2-[3-chlor-4-(1-methoxycarbonyloxyethoxycarbonylmethoxy-phenylacetyl]-
1-Pyrrolidinomethyl-2-[3-chlor-4-(2,3-isopropylidendioxypropoxycarbonylmethoxy)-phenylacetyl]-.

Beispiel 12

Analog Beispiel 1 erhält man mit o-Nitrophenylacetylchlorid das 1-Pyrrolidinomethyl-2-o-nitrophenylacetyl-1,2,3,4-tetrahydroisochinolin, Rf 0,45.

Analog erhält man die nachstehenden 1,2,3,4-Tetrahydroisochinoline:
1-Pyrrolidinomethyl-2-m-nitrophenylacetyl-, Rf 0,72
1-Pyrrolidinomethyl-2-p-nitrophenylacetyl-
(-)-1-Pyrrolidinomethyl-2-m-nitrophenylacetyl- [durch optische Spaltung von 1-Pyrrolidinomethyl-1,2,3,4-tetrahydroisochinolin mit Benzoylweinsäure und Reaktion des (+)-Enantiomeren ([α] + 58°; HCl′, F. 278°) mit m-Nitrophenylacetylchlorid]
(+)-1-Pyrrolidinomethyl-2-m-nitrophenylacetyl-
(-)-1-Pyrrolidinomethyl-2-p-nitrophenylacetyl-, Rf 0.76
(+)-1-Pyrrolidinomethyl-2-p-nitrophenylacetyl-
1-Pyrrolidinomethyl-2-(2-pyridyl-acetyl)-
1-Pyrrolidinomethyl-2-(3-pyridyl-acetyl)-
1-Pyrrolidinomethyl-2-(4-pyridyl-acetyl)-
1-Pyrrolidinomethyl-2-o-(2-oxo-1-imidazolidinyl,-phenylacetyl-
1-Pyrrolidinomethyl-2-m-(2-oxo-1-imidazolidinyl)-phenylacetyl-
1-Pyrrolidinomethyl-2-p-(2-oxo-1-imidazolidinyl)-phenylacetyl-
1-Piperidinomethyl-2-phenylacetyl-, HCl′, F. 217°
1-Piperidinomethyl-2-p-bromphenylacetyl-, HCl′, F. 154°
1-Piperidinomethyl-2-m-tolylacetyl-, HCl′, F. 151°
1-Piperidinomethyl-2-o-nitrophenylacetyl-
1-Piperidinomethyl-2-m-nitrophenylacetyl-
1-Piperidinomethyl-2-p-nitrophenylacetyl-
1-Pyrrolidinomethyl-2-(3-chlor-4-hydroxy-5-nitrophenylacetyl)-, Rf 0,40
1-Pyrrolidinomethyl-2-(3,4-dichlorphenylacetyl)-7-chlor-, Rf 0,95.

Beispiel 13

Analog Beispiel 1 erhält man aus 3-(1-Methylamino-2-pyrrolidinoethyl)-pyridin [erhältlich durch Reaktion von 2-Amino-2-(3-pyridyl)-essigsäure mit Pyrrolidin zum Pyrrolidid, N-Formylierung zu 2-Formamido-2-(3-pyridyl)-essigsäure-pyrrolidid und Reduktion mit $LiAlH_4$] das 3-[1-(N-Methyl-N-3,4-dichlorphenylacetyl-amino)-2-pyrrolidino-ethyl]-pyridin, Rf 0,45.

Analog erhält man aus 2-, 3- oder 4-(1-Methylamino-2-pyrrolidino-ethyl)-pyridin:
2-[1-(N-Methyl-N-o-nitrophenylacetyl-amino)-2-pyrrolidino-ethyl]-pyridin
2-[1-(N-Methyl-N-m-nitrophenylacetyl-amino)-2-pyrrolidino-ethyl]-pyridin
2-[1-(N-methyl-N-p-nitrophenylacetyl-amino)-2-pyrrolidino-ethyl]-pyridin
3-[1(N-Methyl-N-o-nitrophenylacetyl-amino)-2-pyrrolidino-ethyl]-pyridin
3-[1-(N-Methyl-N-m-nitrophenylacetyl-amino)-2-pyrrolidino-ethyl]-pyridin
3-[1-(N-Methyl-N-p-nitrophenylacetyl-amino)-2-pyrrolidino-ethyl]-pyridin

4-[1-(N-Methyl-N-o-nitrophenylacetyl-amino)-2-pyrrolidino-ethyl]-pyridin
4-[1-(N-methyl-N-m-nitrophenylacetyl-amino)-2-pyrrolidino-ethyl]-pyridin
4-[1-(N-Methyl-N-p-nitrophenylacetyl-amino)-2-pyrrolidino-ethyl]-pyridin

Analog erhält man aus 1-Methylamino-1-p-nitrophenyl-2-pyrrolidino-ethan [Di-HCl', F. 212°; erhältlich aus 1-Methylamino-1-phenyl-2-pyrrolidino-ethan über 1-N-Methylacetamido-1-phenyl-2-pyrrolidino-ethan (HCl', F. 240°) und 1-N-Methylacetamido-1-p-nitrophenyl-2-pyrrolidino-ethan (HCl', F. 209°)] das 1-(N-Methyl-N-3,4-dichlorphenylacetylamino)-1-p-nitrophenyl-2-pyrrolidino-ethan.

Beispiel 14

Analog Beispiel 1 erhält man aus 1-Pyrrolidinomethyl-7-nitro-1,2,3,4-tetrahydroisochinolin (erhältlich durch Acetylierung von 1-Pyrrolidinomethyl-1,2,3,4-tetrahydroisochinolin, Nitrierung mit $KNO_3/H_2SO_4$ zu 1-Pyrrolidinomethyl-2-acetyl-7-nitro-1,2,3,4-tetrahydroisochinolin und Abspaltung der Acetylgruppe mit 37%ig. Salzsäure) die nachstehenden 1-Pyrrolidinomethyl-7-nitro-1,2,3,4-tetrahydroisochinoline:
2-(3,4-Dichlorphenylacetyl)-, Rf 0,60
2-m-Methoxyphenylacetyl-, Rf 0,70
2-o-Nitrophenylacetyl-
2-m-Nitrophenylacetyl-
2-p-Nitrophenylacetyl-, Rf 0,63
(-)-2-(3,4-Dichlorphenylacetyl)-, Rf 0,60.

Beispiel 15

Eine Lösung von 10 g 1-Pyrrolidinomethyl-2-o-nitrophenylacetyl-1,2,3,4-tetrahydroisochinolin in 200 ml Methanol wird bei 20° und Normaldruck an 5 g Raney-Ni hydriert, bis die berechnete Menge $H_2$ aufgenommen ist. Man filtriert, dampft das Filtrat ein und erhält 1-Pyrrolidinomethyl-2-o-aminophenylacetyl-1,2,3,4-tetrahydroisochinolin, Rf 0,23.

Analog erhält man durch Reduktion der entsprechenden Nitroverbindungen die nachstehenden 1,2,3,4-Tetrahydroisochinoline:
1-Pyrrolidinomethyl-2-m-aminophenylacetyl-, Rf 0,24
1-Pyrrolidinomethyl-2-p-aminophenylacetyl-
(-)-1-Pyrrolidinomethyl-2-o-aminophenylacetyl-
(+)-1-Pyrrolidinomethyl-2-o-aminophenylacetyl-
(-)-1-Pyrrolidinomethyl-2-m-aminophenylacetyl-
(+)-1-Pyrrolidinomethyl-2-m-aminophenylacetyl-
(-)-1-Pyrrolidinomethyl-2-p-aminophenylacetyl-, Rf 0,53
(+)-1-Pyrrolidinomethyl-2-p-aminophanylacetyl-
1-Pyrrolidinomethyl-2-(3,4-dichlorphenylacetyl)-7-amino-, Rf 0,25
1-Pyrrolidinomethyl-2-m-methoxyphenylacetyl-7-amino-, Rf 0,30
1-Pyrrolidinomethyl-2-o-aminophenylacetyl-7-amino-
1-Pyrrolidinomethyl-2-m-aminophenylacetyl-7-amino-
1-Pyrrolidinomethyl-2-p-aminophenylacetyl-7-amino-, Rf 0,07
(-)-1-Pyrrolidinomethyl-2-(3,4-dichlorphenylacetyl)-7-amino-
1-Piperidinomethyl-2-o-aminophenylacetyl-
1-Piperidinomethyl-2-m-aminophenylacetyl-
1-Piperidinomethyl-2-p-aminophenylacetyl-
1-Pyrrolidinomethyl-2-(3-chlor-4-hydroxy-5-aminophenylacetyl)-, Rf 0,21,
sowie
1-p-Aminophenyl-1-(N-methyl-N-3,4-dichlorphenylacetyl-amino)-2-pyrrolidino-ethan, Rf 0,16
2-[1-(N-Methyl-N-o-aminophenylacetyl-amino)-2-pyrrolidino-ethyl]-pyridin
2-[1-(N-Methyl-N-m-aminophenylacetyl-amino)-2-pyrrolidino-ethyl]-pyridin
2-[1-(N-Methyl-N-p-aminophenylacetyl-amino)-2-pyrrolidino-ethyl]-pyridin
3-[1-(N-Methyl-N-o-aminophenylacetyl-amino)-2-pyrrolidino-ethyl]-pyridin
3-[1-(N-Methyl-N-m-aminophenylacetyl-amino)-2-pyrrolidino-ethyl]-pyridin
3-[1-(N-Methyl-N-p-aminophenylacetyl-amino)-2-pyrrolidino-ethyl]-pyridin
4-[1-(N-Methyl-N-o-aminophenylacetyl-amino)-2-pyrrolidino-ethyl]-pyridin

4-[1-(N-Methyl-N-m-aminophenylacetyl-amino)-2-pyrrolidino-ethyl]-pyridin
4-[1-(N-Methyl-N-p-aminophenylacetyl-amino)-2-pyrrolidino-ethyl]-pyridin.

Beispiel 16

Man löst 3,49 g 1-Pyrrolidinomethyl-2-o-aminophenylacetyl-1,2,3,4-tetrahydroisochinolin in 175 ml $CH_2Cl_2$ und tropft unter Rühren eine Lösung von 0,79 g Acetylchlorid in 10 ml $CH_2Cl_2$ hinzu. Man rührt noch 10 Minuten, konzentriert die Lösung und filtriert das erhaltene 1-Pyrrolidinomethyl-2-o-acetamidophenylacetyl-1,2,3,4-tetrahydroisochinolin ab. Rf 0,22.

Analog erhält man durch Acylierung der entsprechenden primären Aminoverbindungen die nachstehenden 1,2,3,4,-Tetrahydroisochinoline:
1-Pyrrolidinomethyl-2-m-acetamido-phenylacetyl-, Rf 0,29
1-Pyrrolidinomethyl-2-p-acetamido-phenylacetyl-
(-)-1-Pyrrolidinomethyl-2-m-acetamido-phenylacetyl-
(+)-1-Pyrrolidinomethyl-2-m-acetamido-phenylacetyl-
(-)-1-Pyrrolidinomethyl-2-p-acetamido-phenylacetyl-
(+)-1-Pyrrolidinomethyl-2-p-acetamido-phenylacetyl-
1-Pyrrolidinomethyl-2-(3,4-dichlorphenylacetyl)-7-acetamido-, Rf 0,22
1-Pyrrolidinomethyl-2-m-methoxyphenylacetyl-7-acetamido-
1-Pyrrolidinomethyl-2-o-acetamidophenylacetyl-7-acetamido-
1-Pyrrolidinomethyl-2-m-acetamidophenylacetyl-7-acetamido-
1-Pyrrolidinomethyl-2-p-acetamidophenylacetyl-7-acetamido-
(-)-1-Pyrrolidinomethyl-2-(3,4-dichlorphenylacetyl)-7-acetamido-
1-Piperidinomethyl-2-o-acetamido-phenylacetyl-
1-Piperidinomethyl-2-m-acetamido-phenylacetyl-
1-Piperidinomethyl-2-p-acetamido-phenylacetyl-
1-Pyrrolidinomethyl-2-o-methylsulfonamido-phenylacetyl-, Rf 0,40
1-Pyrrolidinomethyl-2-m-methylsulfonamido-phenylacetyl-, Rf 0,32
1-Pyrrolidinomethyl-2-p-methylsulfonamido-phenylacetyl-
1-Piperidinomethyl-2-o-methylsulfonamido-phenylacetyl-
1-Piperidinomethyl-2-m-methylsulfonamido-phenylacetyl-
1-Piperidinomethyl-2-p-methylsulfonamido-phenylacetyl-
sowie
1-p-Acetamidophenyl-1-(N-methyl-N-3,4-dichlorphenylacetyl-amino)-2-pyrrolidino-ethan, Rf 0,15.

Beispiel 17

Ein Gemisch von 1 g 1-Pyrrolidinomethyl-2-o-amino-phenylacetyl-1,2,3,4-tetrahydroisochinolin und 10 ml HCOOH wird 2 Std. gekocht und dann eingedampft. Nach üblicher Aufarbeitung erhält man 1-Pyrrolidinomethyl-2-o-formamido-phenylacetyl-1,2,3,4-tetrahydroisochinolin, Rf 0,30.

Analog erhält man durch Formylierung der entsprechenden primären Amine die nachstehenden 1,2,3,4-Tetrahydroisochinoline:
1-Pyrrolidinomethyl-2-m-formamido-phenylacetyl-, Rf 0,30
1-Pyrrolidinomethyl-2-p-formamido-phenylacetyl-
1-Piperidinomethyl-2-o-formamido-phenylacetyl-
1-Piperidinomethyl-2-m-formamido-phenylacetyl-
1-Piperidinomethyl-2-p-formamido-phenylacetyl-.

Beispiel 18

Man löst 4,22 g 1-Pyrrolidinomethyl-2-o-aminophenylacetyl-1,2,3,4-tetrahydroisochinolin-dihydrochlorid in 50 ml Wasser und versetzt mit 0,81 g KCNO. Nach 3 Std. engt man ein und erhält 1-Pyrrolidinomethyl-2-o-ureido-phenylacetyl-1,2,3,4-tetrahydroisochinolin-HCl; Rf 0,11.

Analog erhält man aus den entsprechenden primären Aminen die nachstehenden 1,2,3,4-Tetrahydroisochinoline:

1-Pyrrolidinomethyl-2-m-ureido-phenylacetyl-, Rf 0,11
1-Pyrrolidinomethyl-2-p-ureido-phenylacetyl-
(-)-1-Pyrrolidinomethyl-2-o-ureido-phenylacetyl-, Rf 0,11; HCl′, [α]-2°
(+)-1-Pyrrolidinomethyl-2-o-ureido-phenylacetyl-
(-)-1-Pyrrolidinomethyl-2-m-ureido-phenylacetyl-, Rf 0,11; HCl′, [α]-77°
(+)-1-Pyrrolidinomethyl-2-m-ureido-phenylacetyl-
(-)-1-Pyrrolidinomethyl-2-p-ureidophenylacetyl-, Rf 0,17; HCl′, [α]-91°
(+)-1-Pyrrolidinomethyl-2-p-ureido-phenylacetyl-
1-Pyrrolidinomethyl-2-(3,4-dichlorphenylacetyl)-7-ureido-, Rf 0,07
1-Pyrrolidinomethyl-2-m-methoxyphenylacetyl-7-ureido-, Rf 0,06
1-Pyrrolidinomethyl-2-o-ureidophenylacetyl-7-ureido-
1-Pyrrolidinomethyl-2-m-ureidophenylacetyl-7-ureido-
1-Pyrrolidinomethyl-2-p-ureidophenylacetyl-7-ureido-
(-)-1-Pyrrolidinomethyl-2-(3,4-dichlorphenylacetyl)-7-ureido-
1-Piperidinomethyl-2-o-ureido-phenylacetyl-
1-Piperidinomethyl-2-o-ureido-phenylacetyl-
1-Piperidinomethyl-2-p-ureido-phenylacetyl-
1-Pyrrolidinomethyl-2-(3-chlor-4-hydroxy-5-ureido-phenylacetyl)-, Rf 0,08
sowie
1-(p-Ureido-phenyl)-1-(N-methyl-N-3,4-dichlorphenylacetyl-amino)-2-pyrrolidino-ethan, Rf 0,06
2-[1-N-Methyl-N-o-ureidophenylacetyl-amino)-2-pyrrolidino-ethyl]-pyridin
2-[1-N-Methyl-N-m-ureidophenylacetyl-amino)-2-pyrrolidino-ethyl]-pyridin
2-[1-N-Methyl-N-p-ureidophenylacetyl-amino)-2-pyrrolidino-ethyl]-pyridin
3-[1-N-methyl-N-o-ureidophenylacetyl-amino)-2-pyrrolidino-ethyl]-pyridin
3-[1-N-Methyl-N-m-ureidophenylacetyl-amino)-2-pyrrolidino-ethyl]-pyridin
3-[1-N-methyl-N-p-ureidophenylacetyl-amino)-2-pyrrolidino-ethyl]-pyridin
4-[1-N-Methyl-N-o-ureidophenylacetyl-amino)-2-pyrrolidino-ethyl]-pyridin
4-[1-N-Methyl-N-m-ureidophenylacetyl-amino)-2-pyrrolidino-ethyl]-pyridin
4-[1-N-Methyl-N-p-ureidophenylacetyl-amino)-2-pyrrolidino-ethyl]-pyridin.

Beispiel 19

Ein Gemisch von 3,49 g 1-Pyrrolidinomethyl-2-o-amino-phenylacetyl-1,2,3,4-tetrahydroisochinolin, 0,57 g Methylisocyanat und 50 ml THF wird 30 Min. bei 20° gerührt.

Man dampft ein, arbeitet wie üblich auf und erhält 1-Pyrrolidinomethyl-2-o-N′-methyl-ureido-phenylacetyl-1,2,3,4-tetrahydroisochinolin, Rf 0,28.

Analog erhält man aus den entsprechenden primären Aminen die nachstehenden 1,2,3,4-Tetrahydroisochinoline:
1-Pyrrolidinomethyl-2-m-N′-methyl-ureido-phenylacetyl-, Rf 0,21
1-Pyrrolidinomethyl-2-p-N′-methyl-ureido-phenylacetyl-
1-Piperidinomethyl-2-o-N′-methyl-ureido-phenylacetyl-
1-Piperidinomethyl-2-m-N′-methyl-ureido-phenylacetyl-
1-Piperidinomethyl-2-p-N′-methyl-ureido-phenylacetyl-.

Beispiel 20

Ein Gemisch von 4,43 g 1-Pyrrolidinomethyl-2-(3-chlor-4-carboxymethoxyphenylacetyl)-1,2,3,4-tetrahydroisochinolin, 1,62 g Carbonyldiimidazol und 140 ml DMF wird 1 Std. bei 20° gerührt. Man gibt 15 ml 25%ige wässerige $NH_3$-Lösung hinzu, rührt weitere 12 Std., dampft ein und nimmt in 1 n wässeriger Salzsäure auf. Man wäscht mit Ethylacetat, arbeitet mit Natronlauge/Ethylacetat wie üblich auf und erhält 1-Pyrrolidinomethyl-2-(3-chlor-4-carbamoylmethoxy-phenylacetyl)-1,2,3,4-tetrahydroisochinolin, Rf 0,19.

Analog erhält man aus den entsprechenden Carboxy-Verbindungen mit $NH_3$ bzw. Methylamin die nachstehenden 1,2,3,4-Tetrahydroisochinoline:
1-Pyrrolidinomethyl-2-o-carbamoyl-phenylacetyl-
1-Pyrrolidinomethyl-2-m-carbamoyl-phenylacetyl-, Rf 0,15
1-Pyrrolidinomethyl-2-p-carbamoyl-phenylacetyl-

1-Pyrrolidinomethyl-2-o-carbamoylmethyl-phenylacetyl-
1-Pyrrolidinomethyl-2-m-carbamoylmethyl-phenylacetyl-, Rf 0,13
1-Pyrrolidinomethyl-2-p-carbamoylmethyl-phenylacetyl-, Rf 0,12
1-Pyrrolidinomethyl-2-o-carbamoylmethoxy-phenylacetyl-
1-Pyrrolidinomethyl-2-m-carbamoylmethoxy-phenylacetyl-
1-Pyrrolidinomethyl-2-p-carbamoylmethoxy-phenylacetyl-
1-Pyrrolidinomethyl-2-o-N-methyl-carbamoyl-phenylacetyl-
1-Pyrrolidinomethyl-2-m-N-methyl-carbamoyl-phenylacetyl-, Rf 0,17
1-Pyrrolidinomethyl-2-p-N-methyl-carbamoyl-phenylacetyl-
1-Pyrrolidinomethyl-2-o-N-methyl-carbamoylmethyl-phenylacetyl-
1-Pyrrolidinomethyl-2-m-N-methyl-carbamoylmethyl-phenylacetyl-
1-Pyrrolidinomethyl-2-p-N-methyl-carbamoylmethyl-phenylacetyl-
1-Pyrrolidinomethyl-2-o-N-methyl-carbamoylmethoxy-phenylacetyl-
1-Pyrrolidinomethyl-2-m-N-methyl-carbamoylmethyl-phenylacetyl-
1-Pyrrolidinomethyl-2-p-N-methyl-carbamoylmethyl-phenylacetyl-
1-Piperidinomethyl-2-o-carbamoyl-phenylacetyl-
1-Piperidinomethyl-2-m-carbamoyl-phenylacetyl-
1-Piperidinomethyl-2-p-carbamoyl-phenylacetyl-
1-Piperidinomethyl-2-o-carbamoylmethyl-phenylacetyl-
1-Piperidinomethyl-2-m-carbamoylmethyl-phenylacetyl-
1-Piperidinomethyl-2-p-carbamoylmethyl-phenylacetyl-
1-Piperidinomethyl-2-o-carbamoylmethoxy-phenylacetyl-
1-Piperidinomethyl-2-m-carbamoylmethoxy-phenylacetyl-
1-Piperidinomethyl-2-p-carbamoylmethoxy-phenylacetyl-
1-Piperidinomethyl-2-o-N-methyl-carbamoyl-phenylacetyl-
1-Piperidinomethyl-2-m-N-methyl-carbamoyl-phenylacetyl-
1-Piperidinomethyl-2-p-N-methyl-carbamoyl-phenylacetyl-
1-Piperidinomethyl-2-o-N-methyl-carbamoylmethyl-phenylacetyl-
1-Piperidinomethyl-2-m-N-methyl-carbamoylmethyl-phenylacetyl-
1-Piperidinomethyl-2-p-N-methyl-carbamoylmethyl-phenylacetyl-
1-Piperidinomethyl-2-o-N-methyl-carbamoylmethoxy-phenylacetyl-
1-Piperidinomethyl-2-m-N-methyl-carbamoylmethoxy-phenylacetyl-
1-Piperidinomethyl-2-p-N-methyl-carbamoylmethoxy-phenylacetyl-.

Beispiel 21

Man tropft 1,23 g Cl-CO-COOCH$_3$ bei 20° unter Rühren zu einer Lösung von 3,49 g 1-Pyrrolidinomethyl-2-o-amino-phenylacetyl-1,2,3,4-tetrahydroisochinolin in 180 ml CH$_2$Cl$_2$. Nach zehnminütigem Nachrühren dampft man ein und löst das erhaltene rohe 1-Pyrrolidinomethyl-2-o-methoxalylaminophenylacetyl-1,2,3,4-tetrahydroisochinolin in 450 ml Methanol. Unter Rühren leitet man bei 20° 15 Minuten lang NH$_3$-Gas ein, läßt 12 Std. stehen, dampft ein und arbeitet wie üblich auf. Man erhält 1-Pyrolidinomethyl-2-o-aminooxalylamino-phenylacetyl-1,2,3,4-tetrahydroisochinolin.

Analog erhält man die nachstehenden 1,2,3,4-Tetrahydroisochinoline:
1-Pyrrolidinomethyl-2-m-methoxalylamino-phenylacetyl-
1-Pyrrolidinomethyl-2-p-methoxalylamino-phenylacetyl-
1-Piperidinomethyl-2-o-methoxalylamino-phenylacetyl-
1-Piperidinomethyl-2-m-methoxalylamino-phenylacetyl-
1-Piperidinomethyl-2-p-methoxalylamino-phenylacetyl-
sowie
1-p-Methoxalylaminophenyl-1-(N-methyl-N-3,4-dichlorphenylacetyl-amino)-2-pyrrolidino-ethan,
und daraus mit NH$_3$ die nachstehenden 1,2,3,4-Tetrahydroisochinoline:
1-Pyrrolidinomethyl-2-m-aminooxalylamino-phenylacetyl-
1-Pyrrolidinomethyl-2-p-aminooxalylamino-phenylacetyl-, Rf 0,47
1-Piperidinomethyl-2-o-aminooxalylamino-phenylacetyl-
1-Piperidinomethyl-2-m-aminooxalylamino-phenylacetyl-
1-Piperidinomethyl-2-p-aminooxalylamino-phenylacetyl-
sowie

1-p-Aminooxalylamino-phenyl-1-(N-methyl-N-3,4-dichlorphenylacetyl-amino)-2-pyrrolidino-ethan, Rf 0,18.

Beispiel 22

Analog Beispiel 21 erhält man aus 1-Pyrrolidinomethyl-2-(3,4-dichlorphenylacetyl)-7-amino-1,2,3,4-tetrahydroisochinolin und Bernsteinsäure-monoethylester-monochlorid das 1-Pyrrolidinomethyl-2-(3,4-dichlorphenylacetyl)-7-(3-ethoxycarbonyl-propionamido)-1,2,3,4-tetrahydroisochinolin (Rf 0,44) und daraus mit $NH_3$ in Methanol das 1-Pyrrolidinomethyl-2-(3,4-dichlorphenylacetyl)-7-(3-carbamoyl-propionamido)-1,2,3,4-tetrahydroisochinolin, Rf 0,05.

Beispiel 23

Analog Beispiel 1 erhält man aus 2-(1-Methylamino-2-pyrrolidono-ethyl)-pyridin und o-Methoxycarbonylphenylacetylchlorid das 2-[1-(N-Methyl-N-o-methoxycarbonyl-phenylacetyl-amino)-2-pyrrolidino-ethyl]-pyridin.

Analog erhält man

2-[1-(N-Methyl-N-m-methoxycarbonyl-phenylacetyl-amino)-2-pyrrolidino-ethyl]-pyridin
2-[1-(N-Methyl-N-p-methoxycarbonyl-phenylacetyl-amino)-2-pyrrolidino-ethyl]-pyridin
3-[1-(N-Methyl-N-o-methoxycarbonyl-phenylacetyl-amino)-2-pyrrolidino-ethyl]-pyridin
3-[1-(N-Methyl-N-m-methoxycarbonyl-phenylacetyl-amino)-2-pyrrolidino-ethyl]-pyridin
3-[1-(N-Methyl-N-p-methoxycarbonyl-phenylacetyl-amino)-2-pyrrolidino-ethyl]-pyridin
4-[1-(N-Methyl-N-o-methoxycarbonyl-phenylacetyl-amino)-2-pyrrolidino-ethyl]-pyridin
4-[1-(N-Methyl-N-m-methoxycarbonyl-phenylacetyl-amino)-2-pyrrolidino-ethyl]-pyridin
4-[1-(N-Methyl-N-p-methoxycarbonyl-phenylacetyl-amino)-2-pyrrolidino-ethyl]-pyridin.

Beispiel 24

Analog Beispiel 10 verseift man den gemäß Beispiel 23 erhaltenen Methylester zu 2-[1-(N-Methyl-N-o-carboxyphenylacetyl-amino)-2-pyrrolidino-ethyl]-pyridin.

Analog erhält man

2-[N-Methyl-N-m-carboxyphenylacetyl-amino)-2-pyrrolidino-ethyl]-pyridin
2-[N-Methyl-N-p-carboxyphenylacetyl-amino)-2-pyrrolidino-ethyl]-pyridin
3-[N-Methyl-N-o-carboxyphenylacetyl-amino)-2-pyrrolidino-ethyl]-pyridin
3-[N-Methyl-N-m-carboxyphenylacetyl-amino)-2-pyrrolidino-ethyl]-pyridin
3-[N-Methyl-N-p-carboxyphenylacetyl-amino)-2-pyrrolidino-ethyl]-pyridin
4-[N-Methyl-N-o-carboxyphenylacetyl-amino)-2-pyrrolidino-ethyl]-pyridin
4-[N-Methyl-N-m-carboxyphenylacetyl-amino)-2-pyrrolidino-ethyl]-pyridin
4-[N-Methyl-N-p-carboxyphenylacetyl-amino)-2-pyrrolidino-ethyl]-pyridin.

Beispiel 25

Analog Beispiel 20 erhält man aus der gemäß Beispiel 24 erhaltenen Carbonsäure das 2-[1-Methyl-N-o-carbamoylphenylacetyl-amino)-2-pyrrolidino-ethyl]-pyridin.

Analog erhält man

2-[1-(N-Methyl-N-m-carbamoylphenylacetyl-amino)-2-pyrrolidino-ethyl]-pyridin
2-[1-(N-Methyl-N-p-carbamoylphenylacetyl-amino)-2-pyrrolidino-ethyl]-pyridin
3-[1-(N-Methyl-N-o-carbamoylphenylacetyl-amino)-2-pyrrolidino-ethyl]-pyridin
3-[1-(N-Methyl-N-m-carbamoylphenylacetyl-amino)-2-pyrrolidino-ethyl]-pyridin
3-[1-(N-Methyl-N-p-carbamoylphenylacetyl-amino)-2-pyrrolidino-ethyl]-pyridin
4-[1-(N-Methyl-N-o-carbamoylphenylacetyl-amino)-2-pyrrolidino-ethyl]-pyridin
4-[1-(N-Methyl-N-m-carbamoylphenylacetyl-amino)-2-pyrrolidino-ethyl]-pyridin
4-[1-(N-Methyl-N-p-carbamoylphenylacetyl-amino)-2-pyrrolidino-ethyl]-pyridin.

Beispiel 26

Analog Beispiel 1 erhält man aus 1-Pyrrolidinomethyl-7-(2-oxo-1-imidazolidinyl)-1,2,3,4-tetrahydroisochinolin [erhältlich aus 1-Pyrrolidinomethyl-7-nitro-1,2,3,4-tetrahydroisochinolin durch N-Benzoylierung und Reduktion zu 1-Pyrrolidinomethyl-2-benzoyl-7-amino-1,2,3,4-tetrahydroisochinolin, aufeinanderfolgende Reaktionen mit Chloracetylchlorid und mit $NH_3$ zu 1-Pyrrolidinomethyl-2-benzoyl-7-aminoacetamido-1,2,3,4-tetrahydroisochinolin, $LiAlH_4$-Reduktion zu 1-Pyrrolidinomethyl- 2-benzyl-7-(2-amino-ethylamino)-1,2,3,4-tetrahydroisochinolin, Reaktion mit Chlorameisensäure-ethylester und hydrogenolytische Abspaltung der Benzylgruppe] das 1-Pyrrolidinomethyl-2-(3,4-dichlorphenylacetyl)-7-(2-oxo-1-imidazolidinyl)-1,2,3,4-tetrahydroisochinolin.

Analog erhält man die nachstehenden 7-(2-Oxo-1-imidazolidinyl)-1,2,3,4-tetrahydroisochinoline:
1-Pyrrolidinomethyl-2-o-nitrophenylacetyl-
1-Pyrrolidinomethyl-2-m-nitrophenylacetyl-
1-Pyrrolidinomethyl-2-p-nitrophenylacetyl-
1-Pyrrolidinomethyl-2-o-methoxyphenylacetyl-
1-Pyrrolidinomethyl-2-m-methoxyphenylacetyl-
1-Pyrrolidinomethyl-2-p-methoxyphenylacetyl-
1-Pyrrolidinomethyl-2-o-(1-naphthyl-acetyl)-
1-Pyrrolidinomethyl-2-o-(4-benzothienyl-acetyl)-.

Beispiel 27

Analog Beispiel 4 erhält man aus 1-p-Toluolsulfonyloxymethyl-2-(3,4-dichlorphenylacetyl)-1,2,3,4-tetrahydroisochinolin und 3-Ureido-pyrrolidin das 1-(3-Ureido-pyrrolidinomethyl)-2-(3,4-dichlorphenylacetyl)-1,2,3,4-tetrahydroisochinolin.

Analog erhält man aus 2-, 3- oder 4-[1-(N-Methyl-N-3,4-dichlorphenylacetyl-amino)-2-brom-ethyl]-pyridin:
2-[1-(N-Methyl-N-3,4-dichlorphenylacetyl-amino)-2-(3-ureido-pyrrolidino)-ethyl]-pyridin
3-[1-(N-Methyl-N-3,4-dichlorphenylacetyl-amino)-2-(3-ureido-pyrrolidino)-ethyl]-pyridin
4-[1-(N-Methyl-N-3,4-dichlorphenylacetyl-amino)-2-(3-ureido-pyrrolidino)-ethyl]-pyridin.

Beispiel 28

Man löst 2,4 g 1-Pyrrolidinomethyl-2-p-acetyl-phenylacetyl-1,2,3,4-tetrahydroisochinolin in 50 ml Ethanol, gibt 1,4 g $NaBH_4$ hinzu und rührt 2 Std. bei 20°. Nach Zersetzung von überschüssigem $NaBH_4$ mit wenig Essigsäure arbeitet man wie üblich auf und enthält 1-Pyrrolidinomethyl-2-p-(1-hydroxyethyl)-phenylacetyl-1,2,3,4-tetrahydroisochinolin, Rf 0,22.

Analog erhält man 1-Pyrrolidinomethyl-2-m-(1-hydroxyethyl)-phenylacetyl-1,2,3,4-tetrahydroisochinolin, Rf 0,24.

Die nachstehenden Beispiele betreffen pharmazeutische Zubereitungen, die Amine der Formel I oder ihre Säureadditionssalze enthalten:

Beispiel A: Tabletten

Ein Gemisch von 0,1 kg "P"-Hydrochlorid, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 1-Pyrrolidinomethyl-2-(1-naphthylacetyl)-1,2,3,4-tetrahydroisochinolin-hydrochlorid werden in üblicher Weise in Hartgelatinekapseln gefüllt, so daß jede Kapsel 20 mg des Wirkstoffs enthält.

Beispiel D: Ampullen

Eine Lösung von 1 kg 1-Pyrrolidinomethyl-2-(4-benzothienylacetyl)-1,2,3,4-tetrahydroisochinolin-hydrochlorid in 15 l 1,2-Propandiol und 15 l zweifach destilliertem Wasser wird steril filtriert, in Ampullen abgefüllt, und steril verschlossen. Jede Ampulle enthält 2 mg Wirkstoff.

Analog sind Tabletten, Dragees, Kapseln und Ampullen erhältlich, die einen oder mehrere der übrigen Wirkstoffe der Formel I und/oder ihrer physiologisch unbedenklichen Salze enthalten.

**Ansprüche**

1. Stickstoffhaltige Ringverbindungen der Formel I

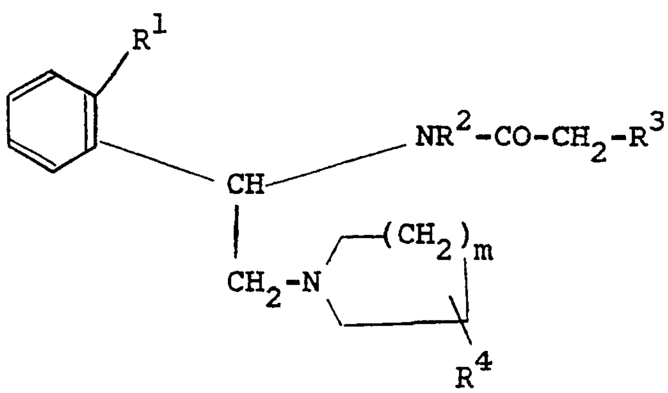

I

worin
$R^1$ H,
$R^2$ A,
$R^1$ und $R^2$ zusammen auch $-(CH_2)_n-$,
$R^3$ eine Phenyl-, Thienyl-, Naphthyl- oder Benzothienylgruppe,
$R^4$ H oder E-Q und
m und n jeweils 1, 2 oder 3
bedeuten,
worin ferner im Benzolring und/oder im Rest $R^3$ unabhängig voneinander jeweils eine CH-Gruppe durch N ersetzt sein kann (können) und/oder worin der Benzolring und/oder der Rest $R^3$ unabhängig voneinander jeweils ein-, zwei- oder dreifach durch A, OA, F, Cl, Br, I, $CF_3$, $NO_2$, $NH_2$, NHA, $NA_2$ und/oder jeweils durch eine Methylendioxygruppe und/oder jeweils durch eine oder zwei der Gruppen -NZ-CY-NZ-und/oder -E-Q substituiert sein kann (können),
worin
E fehlt oder -alkyl-, -O-alkyl-, -NZ-alkyl-, -CY-alkyl-, -NZ-CY-alkyl- oder -CY-NZ-alkyl-,
Q -OH, -NZ-CY-Z, $-NZ-(CY)_r-NHZ$, $-NZ-SO_2-A$, $-COOR^5$, -CO-NH-Z,

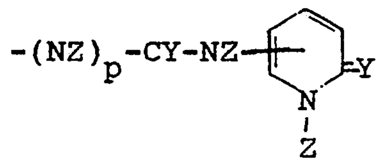
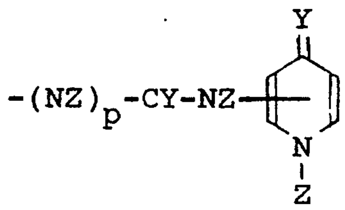,

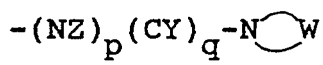 oder $-SO_2-NHZ$,

W $-CH_2CH_2-NZ-CY-$ oder $-CT^1-CT^2-NZ-CT^3-CT^4-$,
Y =O oder =NZ,
Z H oder A,
$T^1$, $T^2$, $T^3$ und $T^4$ jeweils =O oder (H, H),
$R^5$ H, A, AO-alkyl, A-CO-O-alkyl, AO-CO-O-alkyl oder 2-$R^6$O-3-$R^7$O-propyl,
$R^6$ und $R^7$ jeweils A oder zusammen Alkyliden oder Cycloalkyliden mit jeweils bis zu 6-Atomen,
p und q 0 oder 1,
r 1 oder 2,
A Alkyl mit 1-6 C-Atomen und
-alkyl Alkylen mit 1-4 C-Atomen bedeuten,
worin ferner, falls mehrere Gruppen A, E, Q, Y und/oder 2 vorhanden sind, diese jeweils gleich oder verschieden sein können,
sowie deren Salze.

2.
a) 1-Pyrrolidinomethyl-2-(3,4-dichlorphenyl-acetyl)-1,2,3,4-tetrahydro-isochinolin sowie dessen Salze;
b) 1-Pyrrolidinomethyl-2-(1-naphthyl-acetyl)-1,2,3,4-tetrahydro-isochinolin sowie dessen Salze;
c) 1-Pyrrolidinomethyl-2-(4-benzothienyl-acetyl)-1,2,3,4-tetrahydro-isochinolin sowie dessen Salze;
d) 1-Pyrrolidinomethyl-2-o-ureido-phenylacetyl-1,2,3,4-tetrahydro-isochinolin sowie dessen Salze;
e) 1-Pyrrolidinomethyl-2-m-ureido-phenylacetyl-1,2,3,4-tetrahydro-isochinolin sowie dessen Salze;
f) 1-Pyrrolidinomethyl-2-m-carbamoylmethyl-phenylacetyl)-1,2,3,4-tetrahydro-isochinolin sowie dessen Salze;
g) 1-Pyrrolidinomethyl-2-p-carbamoylmethyl-phenylacetyl)-1,2,3,4-tetrahydro-isochinolin sowie dessen Salze;
h) 1-Pyrrolidinomethyl-2-(3,4-dichlorphenylacetyl)-7-ureido-1,2,3,4-tetrahydro-isochinolin sowie dessen Salze.

3. Verfahren zur Herstellung von stickstoffhaltigen Ringverbindungen der Formel I sowie ihrer Salze, dadurch gekennzeichnet, daß man eine Verbindung der Formel II

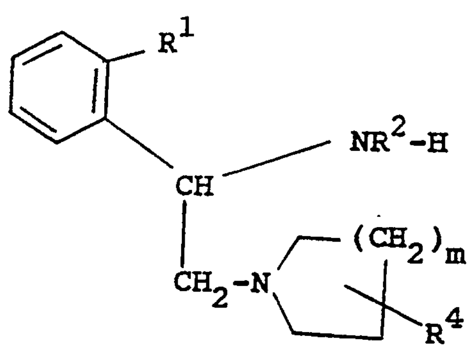
II

worin im Benzolring eine CH-Gruppe durch N ersetzt und/oder der Benzolring wie angegeben substituiert sein kann und $R^1$, $R^2$, $R^4$ und m die angegebenen Bedeutungen haben,
mit einer Verbindung der Formel III
$HOOC-CH_2-R^3$ III

worin $R^3$ die angegebene Bedeutung hat und wie angegeben substituiert sein kann, oder einem ihrer funktionellen Derivate umsetzt
oder daß man eine Verbindung der Formel IV

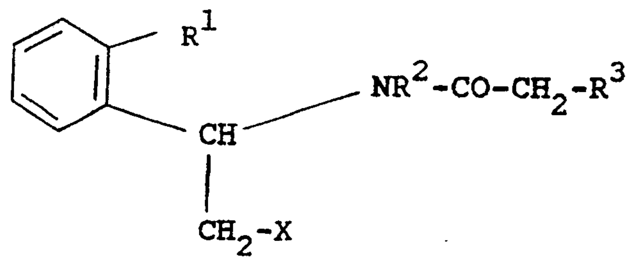

IV

worin im Benzolring und/oder im Rest $R^3$ unabhängig voneinander jeweils eine CH-Gruppe durch N ersetzt sein und/oder der Benzolring und/oder der Rest $R^3$ unabhängig voneinander jeweils wie angegeben substituiert sein kann (können),
X $X^1$ oder $NH_2$ und
$X^1$ Cl, Br, J oder eine freie oder funktionell abgewandelte OH-Gruppe bedeuten und
$R^1$, $R^2$ und $R^3$ die angegebenen Bedeutungen haben,
mit einer Verbindung der Formel V
$X^2$-$CH_2$-$(CH_2)_m$-$CH_2$-$CH_2$-$X^3$ V
worin
ein Wasserstoffatom in einer der $CH_2$-Gruppen durch den Rest $R^4$ ersetzt ist,
$X^2$ und $X^3$ zusammen NH oder, falls X $NH_2$ bedeutet, jeweils $X^1$ bedeuten und
m die angegebene Bedeutung hat,
umsetzt
oder daß man eine Verbindung, die sonst der Formel I entspricht, aber an Stelle eines oder mehrerer H-Atome eine oder mehrere reduzierbare oder hydrogenolytisch abspaltbare Gruppen und/oder C-C- und/oder C-N-Bindungen enthält, mit einem reduzierenden Mittel behandelt,
und/oder daß man in einer Verbindung der Formel I einen oder mehrere der Reste $R^3$, $R^4$, E und/oder Q gegen einen oder mehrere andere Reste $R^3$, $R^4$, E und/oder Q austauscht,
und/oder daß man eine Base bzw. Säure der Formel I durch Behandeln mit einer Säure bzw. Base in eines ihrer Salze überführt.

4. Verfahren zur Herstellung einer pharmazeutischen Zubereitung, dadurch gekennzeichnet, daß man eine Verbindung der Formel I und/oder eines ihrer physiologisch unbedenklichen Salze zusammen mit mindestens einem festen, flüssigen oder halbflüssigen Träger-oder Hilfsstoff in eine pharmazeutische Dosierungsform überführt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I und/oder ihre physiologisch unbedenklichen Salze zur Bekämpfung von Krankheiten.

7. Verwendung einer Verbindung der Formel I und/oder eines ihrer physiologisch unbedenklichen Salze zur Herstellung eines Arzneimittels.

8. Verwendung einer Verbindung der Formel I und/oder eines ihrer physiologisch unbedenklichen Salze bei der Bekämpfung von Krankheiten.